# EUROPEAN PATENT APPLICATION

(11) **EP 4 624 497 A1**
(43) Date of publication of application: **01.10.2025**
(21) Application number: 23893907.8
(22) Date of filing: 22.11.2023
(51) Int. Cl.: C07K 19/00, C12N 15/62, C12N 15/63, C12N 5/10, G01N 33/68, G01N 33/58

(54) **PHOSPHOENOLPYRUVATE OPTICAL PROBE, PREPARATION METHOD THEREFOR AND APPLICATION THEREOF**

(30) Priority: 22.11.2022 CN 202211465765
(71) Applicant: East China University of Science and Technology, Shanghai 200237 (CN)
(72) Inventor: YANG, Yi, Shanghai 200237 (CN); ZHAO, Yuzheng, Shanghai 200237 (CN); SUN, Yuying, Shanghai 200237 (CN); LI, Xie, Shanghai 200237 (CN); ZOU, Yejun, Shanghai 200237 (CN)
(74) Representative: Nederlandsch Octrooibureau
(86) International application number: PCT/CN2023/133332
(87) International publication number: WO 2024/109819

(57) **Abstract**

The present invention relates to a phosphoenolpyruvate optical probe. Specifically, the present invention provides a phosphoenolpyruvate optical probe, which comprises a phosphoenolpyruvate-sensitive peptide and an optically active peptide, wherein the optically active polypeptide is located within a sequence of the phosphoenolpyruvate-sensitive polypeptide.

## Description

This application claims priority to Chinese patent application No.202211465765.3, filed on November 22, 2022, the entirety of which is hereby incorporated by reference for all purposes herein.

### TECHNICAL FIELD

The present invention relates to the field of optical probes technology, and in particular to a phosphoenolpyruvate optical probe and a preparation method and application thereof.

### BACKGROUND

Phosphoenolpyruvate (Phosphoenolpyruvate, PEP), as a high-energy intermediate in the glycolysis pathway, is a key metabolite connecting glycolysis and gluconeogenesis. The high-energy phosphate bonds contained in PEP enable it to play the role of a phosphate donor in bacteria, plants, and mammalian cells, and play an important role in maintaining intracellular ATP content, glucose homeostasis, carbohydrate metabolism regulation, tumor immunity, and cellular protection

Currently, the most commonly used methods for detecting PEP include high performance liquid chromatography, liquid chromatography-mass spectrometry (Ho P C et al. Cell, 2015, 162(6), 1217-1228), isotope labeling (Heiden MG V et al. Science, 2010, 329(5998), 1492-1499) and enzyme activity detection methods. High performance liquid chromatography and liquid chromatography-mass spectrometry can efficiently and accurately measure the changes in the content of PEP in samples, but it involves the extraction of intracellular metabolites, and the experimental steps are cumbersome and the cycle is long. Neither isotope labeling nor enzyme activity detection can monitor the changes of PEP in living cells in real time, so it is urgent to develop a genetically encoded fluorescent probe that can monitor the dynamic changes of PEP in living cells in situ.

### SUMMARY

The object of the present invention is to provide probes and methods for real-time localization, high-throughput and quantitative detection of phosphoenolpyruvate inside and outside cells.

To achieve the object of the invention, the invention provides the following technical solutions:
The first aspect of the present invention provides a phosphoenolpyruvate binding protein variant:
(a) having the sequence shown in SEQ ID NO:1 and having mutations at 1, 2, 3, 4, 5, 6, 7, 8 or more sites selected from the group consisting of: I253, Q254, R255, G256, G257, R21, V54, R55, V57, A58, N59, I60, R72, R212, G213, K215, S216, L320, wherein the mutations comprise modifications, substitutions or deletions of amino acids, or
(b) being a sequence having at least 70% sequence identity with (a) and having the mutation described in (a) and retaining the ability to bind to phosphoenolpyruvate.

In one or more embodiments, the phosphoenolpyruvate binding protein variant has the sequence shown in SEQ ID NO: 1, and: (I) has mutations at 1, 2, 3, 4 or more sites selected from the group consisting of: I253, Q254, R255, G256, G257; and/or, (II) has mutations at 1, 2, 3, 4, 5 or more sites selected from the group consisting of: R21, V54, R55, V57, A58, N59, I60, R72, R212, G213, K215, S216, L320.

In one or more embodiments, the mutation sites in (I) comprise Q254. Preferably, the mutation sites in (I) comprise sites selected from any one or more groups consisting of: (1.1) I253, Q254, (1.2) I253, Q254, R255, G256, (1.3) Q254, R255, G256, (1.4) I253, Q254, G256, G257.

In one or more embodiments, the mutation sites in (II) comprise sites selected from any one or more groups consisting of:(11.1) R21, (II.2) V54, (II.3) R55, (II.4) V57, (II.5) A58, (II.6) I60, (II.7) N59, (II.8) R212, (II.9) G213, (II.10) K215, (II.11) S216, (II.12) L320, (II.13) R72, (II.14) V54, R72.

In one or more embodiments, the phosphoenolpyruvate binding protein variant has the sequence shown in SEQ ID NO: 1, and: (I) has mutations at the following sites: I253, Q254, G256, and G257, and (II) has mutations at sites selected from any one or more groups consisting of: (II.1) R21, (II.2) V54, (II.3) R55, (II.4) V57, (II.5) A58, (II.6) I60, (II.7) N59, (II.8) R212, (II.9) G213, (II.10) K215, (II.11) S216, (II.12) L320, (II.13) R72, (II.14) V54, R72.

In one or more embodiments, the phosphoenolpyruvate binding protein variant has the sequence shown in SEQ ID NO: 1, and has mutations at the following sites: (II.15) V54, 1253, Q254, R255, G256, (II.16) N59, 1253, Q254, R255, G256, (II.17) V54, 1253, Q254, G256, G257, (II.18) N59, 1253, Q254, G256, G257, (II.19) R212, I253, Q254, G256, G257, (II.20) G213, I253, Q254, G256, G257, (II.21) K215, I253, Q254, G256, G257, (II.22) L320, 1253, Q254, G256, G257, (II.23) S216, I253, Q254, G256, G257.

In one or more embodiments, I253 is mutated to V, N, F, C, E, G, S, K, H, D, P, L, R, T or Q.

In one or more embodiments, Q254 is mutated to L, V, A, R, P, M, K, or I.

In one or more embodiments, R255 is mutated to L, I, or F.

In one or more embodiments, G256 is mutated to H, F, Y, or V.

In one or more embodiments, R21 is mutated to K.

In one or more embodiments, V54 is mutated to G, K, or Q.

In one or more embodiments, R55 is mutated to S.

In one or more embodiments, V57 is mutated to M.

In one or more embodiments, A58 is mutated to G.

In one or more embodiments, N59 is mutated to D, H or E.

In one or more embodiments, I60 is mutated to W.

In one or more embodiments, R72 is mutated to A.

In one or more embodiments, R212 is mutated to I, E, or M.

In one or more embodiments, G213 is mutated to T.

In one or more embodiments, K215 is mutated to H, D, or E.

In one or more embodiments, S216 is mutated to D, L, Y, I, M, F or Q.

In one or more embodiments, L320 is mutated to Y, N or F.

In one or more embodiments, the mutations comprise one or more selected from the group consisting of: I253K, Q254L, R255L and/or G256F.

In one or more embodiments, the mutations comprise those selected from any one group consisting of: (1) I253Y, Q254I, (2) I253V, Q254L, R255L, G256H, (3) Q254L, R255L, G256H, (4) Q254V, R2551, G256H, (5) I253N, Q254L, R255L, G256F, (6) I253F, Q254L, R255L, G256F, (7) I253V, Q254A, R255F, G256Y, (8) I253V, Q254L, R255F, G256Y, (9) I253C, Q254L, R255L, G256F, (10) I253E, Q254L, R255L, G256F, (11) I253G, Q254L, R255L, G256F, (12) I253I, Q254L, R255L, G256F, (13) I253S, Q254L, R255L, G256F, (14) I253G, Q254L, R255L, G256F, (15) I253K, Q254L, R255L, G256F, (16) I253H, Q254L, R255L, G256F, (17) I253D, Q254L, R255L, G256F, (18) Q254L, R255L, G256F, (19) I253P, Q254L, R255L, G256F, (20) Q254L, R255L, G256F, (21) I253L, Q254R, R255L, G256F, (22) I253R, Q254L, R255L, G256F, (23) I253T, Q254L, R255F, G256Y, (24) I253Q, Q254P, R255F, G256Y, (25) I253Q, Q254M, R255F, G256Y, (26) I253T, Q254P, R255F, G256Y, (27) Q254L, R255F, G256F, (28) I253S, Q254K, R255F, G256F, (29) Q254P, R255F, G256F, (30) I253E, Q254V, R255F, G256F, (31) Q254L, R255F, G256F, (32) I253T, Q254L, R255F, G256F, (33) Q254L, R255F, G256F, (34) I253D, Q254L, G256V, G257L, (35) I253S, Q254L, G256V, G257L, (36) I253Q, Q254L, G256V, G257L, (37) R21K, (38) V54K, (39) V54Q, (40) V54G, (41) R55S, (42) V57M, (43) A58G, (44) I60W, (45) N59D, (46) N59H, (47) N59E, (48) R2121, (49) R212E, (50) R212M, (51) G213T, (52) K215E, (53) K215H, (54) K215D, (55) S216D, (56) S216L, (57) S216Y, (58) S2161, (59) S216M, (60) S216F, (61) S216Q, (62) L320Y, (63) L320N, (64) L320F, (65) R72A, (66) V54G, R72A, (67) V54K, I253G, Q254L, R255L, G256F, (68) V54Q, I253G, Q254L, R255L, G256F, (69) N59H, I253G, Q254L, R255L, G256F, (70) V54K, I253K, Q254L, R255L, G256F, (71) V54Q, I253K, Q254L, R255L, G256F, (72) N59H, I253K, Q254L, R255L, G256F, (73) V54Q, I253Q, Q254L, G256V, G257L, (74) N59D, I253Q, Q254L, G256V, G257L, (75) R212M, I253Q, Q254L, G256V, G257L, (76) G213T, I253Q, Q254L, G256V, G257L, (77) K215E, I253Q, Q254L, G256V, G257L, (78) L320F, I253Q, Q254L, G256V, G257L, (79) S216L, I253Q, Q254L, G256V, G257L, (80) S216Y, I253Q, Q254L, G256V, G257L.

The first aspect of the present invention further provides a phosphoenolpyruvate optical probe, comprising a phosphoenolpyruvate-sensitive polypeptide and an optically active polypeptide, wherein the optically active polypeptide is located in the sequence of the phosphoenolpyruvate-sensitive polypeptide. The phosphoenolpyruvate-sensitive polypeptide is divided into a first part and a second part by the optically active polypeptide.

In one or more embodiments, the phosphoenolpyruvate-sensitive polypeptide has:
(1) the sequence of SEQ ID NO: 1, or a sequence having at least 70% sequence identity with it and retaining the binding activity to phosphoenolpyruvate,
(2) a functional variant of the sequence of SEQ ID NO: 1, which has mutations within 5 (preferably 3, more preferably 2) amino acids from the junction with the optically active polypeptide, and/or has mutations at 1, 2, 3, 4, 5 or more sites selected from the group consisting of: R21, V54, R55, V57, A58, N59, I60, R72, R212, G213, K215, S216, L320,
(3) a sequence having at least 70% sequence identity to the sequence described in (2), having the mutations described in (2), and retaining the sensitivity to phosphoenolpyruvate.

In one or more embodiments, (2) is the sequence shown in SEQ ID NO: 1 and has mutations at 1, 2, 3, 4, 5 or more positions selected from the group consisting of: I253, Q254, R255, G256, G257, R21, V54, R55, V57, A58, N59, I60, R72, R212, G213, K215, S216, L320, wherein the mutations comprise modifications, substitutions or deletions of amino acids.

In one or more embodiments, (2) is the sequence of the phosphoenolpyruvate binding protein variant described in any embodiment of the first aspect of the present invention.

In one or more embodiments, the optically active polypeptide is located at or replaces residues 68-71, 168-173 and/or 253-257 of the phosphoenolpyruvate-sensitive polypeptide, and the numbering corresponds to the full length of the phosphoenolpyruvate-sensitive polypeptide.

In one or more embodiments, the optically active polypeptide is located at any one or more of the following positions of the phosphoenolpyruvate-sensitive polypeptide: 68/69, 68/70, 69/70, 68/71, 69/71, 70/71, 168/169, 168/170, 169/170, 168/171, 169/171, 170/171, 168/172, 169 /172, 170/172, 171/172, 168/173, 169/173, 170/173, 171/173, 172/173, 253/254, 253/255, 254/255, 253/256, 254/256, 255/256, 253/257, 254/257, 255/257 and/or 256/257. More preferably, the optically active polypeptide is located at any one or more of the following sites of the phosphoenolpyruvate-sensitive polypeptide: 69/71, 170/171, 171/172, 254/255, 254/256 and 255/257.

In one or more embodiments, the optically active polypeptide is a fluorescent protein or a functional variant thereof, wherein the functional variant of the fluorescent protein has mutations within 3 (preferably 2) amino acids from the junction with the optically active polypeptide.

In one embodiment, the fluorescent protein is selected from the group consisting of yellow fluorescent protein, orange fluorescent protein, red fluorescent protein, green fluorescent protein, blue fluorescent protein, and apple red fluorescent protein. In one embodiment, the fluorescent protein has a sequence as shown in any one of SEQ ID NO: 2-9, preferably SEQ ID NO: 2, 6, 7 or 9.

In one or more embodiments, the functional variant of the fluorescent protein has a sequence as shown in any one of SEQ ID NO: 2-9 and has a mutation at the first amino acid corresponding to SEQ ID NO: 2, and the mutation is preferably mutated to S, G, N, T, P, A, E, or H. Alternatively or in addition, the functional variant of the fluorescent protein has a sequence as shown in any one of SEQ ID NO: 2-9 and has a mutation at the amino acid 246 corresponding to SEQ ID NO: 2, and the mutation is preferably mutated to F, C, W, or H.

In one or more embodiments, the functional variant of the fluorescent protein has a sequence as shown in any one of SEQ ID NO: 2-9 and has mutations at the first amino acid and the amino acid 246 corresponding to SEQ ID NO: 2 selected from any one of the following groups: 1S and 246F; 1G and 246F; 1H and 246F; 1A and 246C; 1G and 246C; 1S and 246C; 1T and 246C; 1T and 246F.

In one or more embodiments, the fluorescent protein has the sequence shown in SEQ ID NO: 2 or is a variant with mutations at position 1 and/or position 246 as shown in any one of the following groups: Y1S; Y1G; Y1N; Y1T; Y1P; Y1A; Y1E; Y1H; N246F; N246C; N246W; N246H; Y1S and N246F; Y1G and N246F; Y1H and N246F; Y1A and N246C; Y1G and N246C; Y1S and N246C; Y1T and N246C; Y1T and N246F. The optically active polypeptide is located at one or more sites of the phosphoenolpyruvate-sensitive polypeptide selected from the group consisting of: 69/71, 170/171, 171/172, 254/255, 254/256, and 255/257 sites.

In one or more embodiments, the fluorescent protein has the sequence shown in SEQ ID NO: 6 or is a variant that has mutations at the amino acids corresponding to amino acid 1 or amino acid 246 of SEQ ID NO: 2 as shown in any of the following groups: 1S; 1G; 1N; 1T; 1P; 1A; 1E; 1H; 246F; 246C; 246W; 246H; 1S and 246F; 1G and 246F; 1H and 246F; 1A and 246C; 1G and 246C; 1S and 246C; 1T and 246C; 1T and 246F. The optically active polypeptide is located at one or more sites of the phosphoenolpyruvate-sensitive polypeptide selected from the group consisting of: 171/172, 254/255, and 254/256 sites.

In one or more embodiments, the fluorescent protein has the sequence shown in SEQ ID NO: 7 or is a variant that has mutations at the amino acids corresponding to amino acid 1 or amino acid 246 of SEQ ID NO: 2 as shown in any of the following groups: 1S; 1G; 1N; 1T; 1P; 1A; 1E; 1H; 246F; 246C; 246W; 246H; 1S and 246F; 1G and 246F; 1H and 246F; 1A and 246C; 1G and 246C; 1S and 246C; 1T and 246C; 1T and 246F. The optically active polypeptide is located at one or more sites of the phosphoenolpyruvate-sensitive polypeptide selected from the group consisting of: 170/171, 171/172, 254/255, and 254/256 sites.

In one or more embodiments, the fluorescent protein has the sequence shown in SEQ ID NO: 10 or is a variant that has mutations at the amino acids corresponding to amino acid 1 or amino acid 246 of SEQ ID NO: 2 as shown in any of the following groups: 1S; 1G; 1N; 1T; 1P; 1A; 1E; 1H; 246F; 246C; 246W; 246H; 1S and 246F; 1G and 246F; 1H and 246F; 1A and 246C; 1G and 246C; 1S and 246C; 1T and 246C; 1T and 246F. The optically active polypeptide is located at one or more sites of the phosphoenolpyruvate-sensitive polypeptide selected from the group consisting of: 170/171, 171/172, 254/255, and 254/256 sites.

In one embodiment, the optical probe further comprises one or more linkers flanking the optically active polypeptide. The linker described in the present invention can be any amino acid sequence of any length. In one embodiment, the flanks of the optically active polypeptide comprise linkers of no more than 5 amino acids, such as linkers of 0, 1, 2, 3, or 4 amino acids. In one embodiment, the linker flanking the optically active polypeptide contains the amino acid Y. In one embodiment, the linker Y is located at the N-terminus and/or C-terminus of the optically active polypeptide. In one embodiment, the optical probe is as follows: the first part B1 of the phosphoenolpyruvate-sensitive polypeptide, Y, the optically active polypeptide A, and the second part B2 of the phosphoenolpyruvate-sensitive polypeptide. In one embodiment, the optical probe of the present invention does not contain a linker.

In one embodiment, the optical probe of the present invention further includes a targeting sequence, which is used to target the probe to specific organelles in a cell, for instance.

In one or more embodiments, in the optical probe, the phosphoenolpyruvate-sensitive polypeptide is as shown in SEQ ID NO: 1, and the optically active polypeptide is as shown in SEQ ID NO: 2-9 (preferably SEQ ID NO: 2, 6, 7, 9) or is a variant having one or more mutations at the amino acids corresponding to amino acid 1 or amino acid 246 of SEQ ID NO: 2 selected from the group consisting of: 1S, 1G, 1N, 1T, 1P, 1A, 1E, 1H, 246F, 246C, 246W, or 246H. The optically active polypeptide is located at any one or more sites of the phosphoenolpyruvate-sensitive polypeptide: 68/69, 68/70, 69/70, 68/71, 69/71, 70/71, 168/169, 168/170, 169/170, 168/171, 169/171, 170/171, 168/172, 169/172, 170/172, 171/172, 168/173, 169/173, 170/173, 171/173, 172/173, 253/254, 253/255, 254/255, 253/256, 254/256, 255/256, 253/257, 254/257, 255/257 and/or 256/257. Preferably, the optically active polypeptide is located at any one or more of the following sites of the phosphoenolpyruvate-sensitive polypeptide selected from the group consisting of: 69/71, 170/171, 171/172, 254/255, 254/256, and 255/257.

In one or more embodiments, in the optical probe, the phosphoenolpyruvate-sensitive polypeptide is as shown in SEQ ID NO: 1 and has one or more of the following mutations: I253V, I253N, I253F, I253C, I253E, I253G, I253S, I253K, I253H, I253D, I253P, I253L, I253R, I253T, I253Q, Q254L, Q254V, Q254A, Q254R, Q254P, Q254M, Q254K, Q2541, R255L, R2551, R255F, G256H, G256F, G256Y, G256V, G257L, R21K, V54G, V54K, V54Q, R55S, V57M, A58G, N59D, N59H, N59E, I60W, R72A, R2121, R212E, R212M, G213T, K215H, K215D, K215E, S216D, S216L, S216Y, S2161, S216M, S216F, S216Q, L320Y, L320N and/or L320F, the optically active polypeptide is as shown in SEQ ID NOs: 2-9 (preferably SEQ ID NO: 2, 6, 7, 9) or is a variant having one or more mutations selected from the following at the amino acids corresponding to amino acid 1 or amino acid 246 of SEQ ID NO: 2: 1S, 1G, 1N, 1T, 1P, 1A, 1E, 1H, 246F, 246C, 246W or 246H, the optically active polypeptide is located at the 254/255 or 254/256 site of the phosphoenolpyruvate-sensitive polypeptide. Preferably, the mutations of the phosphoenolpyruvate-sensitive polypeptide comprise mutations selected from any of the group consisting of: (1) I253Y, Q2541, (2) I253V, Q254L, R255L, G256H, (3) Q254L, R255L, G256H, (4) Q254V, R255I, G256H, (5) I253N, Q254L, R255L, G256F, (6) I253F, Q254L, R255L, G256F, (7) I253V, Q254A, R255F, G256Y, (8) I253V, Q254L, R255F, G256Y, (9) I253C, Q254L, R255L, G256F, (10) I253E, Q254L, R255L, G256F, (11) I253G, Q254L, R255L, G256F, (12) I253I, Q254L, R255L, G256F, (13) I253S, Q254L, R255L, G256F, (14) I253G, Q254L, R255L, G256F, (15) I253K, Q254L, R255L, G256F, (16) I253H, Q254L, R255L, G256F, (17) I253D, Q254L, R255L, G256F, (18) Q254L, R255L, G256F, (19) I253P, Q254L, R255L, G256F, (20) Q254L, R255L, G256F, (21) I253L, Q254R, R255L, G256F, (22) I253R, Q254L, R255L, G256F, (23) I253T, Q254L, R255F, G256Y, (24) I253Q, Q254P, R255F, G256Y, (25) I253Q, Q254M, R255F, G256Y, (26) I253T, Q254P, R255F, G256Y, (27) Q254L, R255F, G256F, (28) I253S, Q254K, R255F, G256F, (29) Q254P, R255F, G256F, (30) I253E, Q254V, R255F, G256F, (31) Q254L, R255F, G256F, (32) I253T, Q254L, R255F, G256F, (33) Q254L, R255F, G256F, (34) I253D, Q254L, G256V, G257L, (35) I253S, Q254L, G256V, G257L, (36) I253Q, Q254L, G256V, G257L, (37) R21K, (38) V54K, (39) V54Q, (40) V54G, (41) R55S, (42) V57M, (43) A58G, (44) I60W, (45) N59D, (46) N59H, (47) N59E, (48) R212I, (49) R212E, (50) R212M, (51) G213T, (52) K215E, (53) K215H, (54) K215D, (55) S216D, (56) S216L, (57) S216Y, (58) S216I, (59) S216M, (60) S216F, (61) S216Q, (62) L320Y, (63) L320N, (64) L320F, (65) R72A, (66) V54G, R72A, (67) V54K, I253G, Q254L, R255L, G256F, (68) V54Q, I253G, Q254L, R255L, G256F, (69) N59H, I253G, Q254L, R255L, G256F, (70) V54K, I253K, Q254L, R255L, G256F, (71) V54Q, I253K, Q254L, R255L, G256F, (72) N59H, I253K, Q254L, R255L, G256F, (73) V54Q, I253Q, Q254L, G256V, G257L, (74) N59D, I253Q, Q254L, G256V, G257L, (75) R212M, I253Q, Q254L, G256V, G257L, (76) G213T, I253Q, Q254L, G256V, G257L, (77) K215E, I253Q, Q254L, G256V, G257L, (78) L320F, I253Q, Q254L, G256V, G257L, (79) S216L, I253Q, Q254L, G256V, G257L, (80) S216Y, I253Q, Q254L, G256V, G257L.

In one or more embodiments, in the optical probe, the phosphoenolpyruvate-sensitive polypeptide is as shown in SEQ ID NO: 1, the optically active polypeptide is as shown in SEQ ID NOs: 2-9 (preferably SEQ ID NOs: 2, 6, 7, 9), the optically active polypeptide is located at the 254/255 site of the phosphoenolpyruvate-sensitive polypeptide, and the optical probe has the following mutations: (1) I253Y and Q254I of the phosphoenolpyruvate-sensitive polypeptide, (2) I253V, Q254L, R255L, G256H of the phosphoenolpyruvate-sensitive polypeptide and 1S of the optically active polypeptide, (3) Q254L, R255L, G256H of the phosphoenolpyruvate-sensitive polypeptide and 1S of the optically active polypeptide, (4) Q254V, R2551, G256H of the phosphoenolpyruvate-sensitive polypeptide and 246W of the optically active polypeptide, (5) I253N, Q254L, R255L, G256F of the phosphoenolpyruvate-sensitive polypeptide and 1S, 246F of the optically active polypeptide, (6) I253F, Q254L, R255L, G256F of the phosphoenolpyruvate-sensitive polypeptide and 1G, 246F of the optically active polypeptide, (7) I253V, Q254A, R255F, G256Y of the phosphoenolpyruvate-sensitive polypeptide and 246F of the optically active polypeptide, (8) I253V, Q254L, R255F, G256Y of the phosphoenolpyruvate-sensitive polypeptide and 1H, 246F of the optically active polypeptide, (9) I253C, Q254L, R255L, G256F of the phosphoenolpyruvate-sensitive polypeptide and 1N of the optically active polypeptide, (10) I253E, Q254L, R255L, G256F of the phosphoenolpyruvate-sensitive polypeptide and 1S of the optically active polypeptide, (11) I253G, Q254L, R255L, G256F of the phosphoenolpyruvate-sensitive polypeptide and 1T of the optically active polypeptide, (12) I253I, Q254L, R255L, G256F of the phosphoenolpyruvate-sensitive polypeptide and 1S of the optically active polypeptide, (13) I253S, Q254L, R255L, G256F of the phosphoenolpyruvate-sensitive polypeptide and 1S of the optically active polypeptide, (14) I253G, Q254L, R255L, G256F of the phosphoenolpyruvate-sensitive polypeptide and 1P of the optically active polypeptide, (15) I253K, Q254L, R255L, G256F of the phosphoenolpyruvate-sensitive polypeptide and 1S of the optically active polypeptide, (16) I253H, Q254L, R255L, G256F of the phosphoenolpyruvate-sensitive polypeptide and 1N of the optically active polypeptide, (17) I253D, Q254L, R255L, G256F of the phosphoenolpyruvate-sensitive polypeptide and 1S of the optically active polypeptide, (18) Q254L, R255L, G256F of the phosphoenolpyruvate-sensitive polypeptide and 1S of the optically active polypeptide, (19) I253P, Q254L, R255L, G256F of the phosphoenolpyruvate-sensitive polypeptide and 1T of the optically active polypeptide, (20) Q254L, R255L, G256F of the phosphoenolpyruvate-sensitive polypeptide and 1T of the optically active polypeptide, (21) I253L, Q254R, R255L, G256F of the phosphoenolpyruvate-sensitive polypeptide, (22) I253R, Q254L, R255L, G256F of the phosphoenolpyruvate-sensitive polypeptide and 1S of the optically active polypeptide, (23) I253T, Q254L, R255F, G256Y of the phosphoenolpyruvate-sensitive polypeptide and 1T of the optically active polypeptide, (24) I253Q, Q254P, R255F, G256Y of the phosphoenolpyruvate-sensitive polypeptide, (25) I253Q, Q254M, R255F, G256Y of the phosphoenolpyruvate-sensitive polypeptide and 1P of the optically active polypeptide, (26) I253T, Q254P, R255F, G256Y of the phosphoenolpyruvate-sensitive polypeptide and 1A of the optically active polypeptide, (27) Q254L, R255F, G256F of the phosphoenolpyruvate-sensitive polypeptide and 1N of the optically active polypeptide, (28) I253S, Q254K, R255F, G256F of the phosphoenolpyruvate-sensitive polypeptide and 1E of the optically active polypeptide, (29) Q254P, R255F, G256F of the phosphoenolpyruvate-sensitive polypeptide and 246C of the optically active polypeptide, (30) I253E, Q254V, R255F, G256F of the phosphoenolpyruvate-sensitive polypeptide and 1A, 246C of the optically active polypeptide, (31) Q254L, R255F, G256F of the phosphoenolpyruvate-sensitive polypeptide and 1G, 246C of the optically active polypeptide, (32) I253T, Q254L, R255F, G256F of the phosphoenolpyruvate-sensitive polypeptide and 1S, 246C of the optically active polypeptide, (33) Q254L, R255F, G256F of the phosphoenolpyruvate-sensitive polypeptide and 1T, 246C of the optically active polypeptide, (34) R21K of the phosphoenolpyruvate-sensitive polypeptide, (35) V54K of the phosphoenolpyruvate-sensitive polypeptide, (36) V54Q of the phosphoenolpyruvate-sensitive polypeptide, (37) V54G of the phosphoenolpyruvate-sensitive polypeptide, (38) R55S of the phosphoenolpyruvate-sensitive polypeptide, (39) V57M of the phosphoenolpyruvate-sensitive polypeptide, (40) A58G of the phosphoenolpyruvate-sensitive polypeptide, (41) I60W of the phosphoenolpyruvate-sensitive polypeptide, (42) N59D of the phosphoenolpyruvate-sensitive polypeptide, (43) N59H of the phosphoenolpyruvate-sensitive polypeptide, (44) N59E of the phosphoenolpyruvate-sensitive polypeptide, (45) R212I of the phosphoenolpyruvate-sensitive polypeptide, (46) R212E of the phosphoenolpyruvate-sensitive polypeptide, (47) R212M of the phosphoenolpyruvate-sensitive polypeptide, (48) G213T of the phosphoenolpyruvate-sensitive polypeptide, (49) K215E of the phosphoenolpyruvate-sensitive polypeptide, (50) K215H of the phosphoenolpyruvate-sensitive polypeptide, (51) K215D of the phosphoenolpyruvate-sensitive polypeptide, (52) S216D of the phosphoenolpyruvate-sensitive polypeptide, (53) S216L of the phosphoenolpyruvate-sensitive polypeptide, (54) S216Y of the phosphoenolpyruvate-sensitive polypeptide, (55) S2161 of the phosphoenolpyruvate-sensitive polypeptide, (56) S216M of the phosphoenolpyruvate-sensitive polypeptide, (57) S216F of the phosphoenolpyruvate-sensitive polypeptide, (58) S216Q of the phosphoenolpyruvate-sensitive polypeptide, (59) L320Y of the phosphoenolpyruvate-sensitive polypeptide, (60) L320N of the phosphoenolpyruvate-sensitive polypeptide, (61) L320F of the phosphoenolpyruvate-sensitive polypeptide, (62) R72A of the phosphoenolpyruvate-sensitive polypeptide, (63) V54G, R72A of the phosphoenolpyruvate-sensitive polypeptide, (64) V54K, I253G, Q254L, R255L, G256F of the phosphoenolpyruvate-sensitive polypeptide and 1P of the optically active polypeptide, (65) V54Q, I253G, Q254L, R255L, G256F of the phosphoenolpyruvate-sensitive polypeptide and 1P of the optically active polypeptide, (66) N59H, I253G, Q254L, R255L, G256F of the phosphoenolpyruvate-sensitive polypeptide and 1P of the optically active polypeptide, (67) V54K, I253K, Q254L, R255L, G256F of the phosphoenolpyruvate-sensitive polypeptide and 1S of the optically active polypeptide, (68) V54Q, I253K, Q254L, R255L, G256F of the phosphoenolpyruvate-sensitive polypeptide and 1S of the optically active polypeptide, (69) N59H, I253K, Q254L, R255L, G256F of the phosphoenolpyruvate-sensitive polypeptide and 1S of the optically active polypeptide.

In one or more embodiments, in the optical probe, the phosphoenolpyruvate-sensitive polypeptide is as shown in SEQ ID NO: 1, the optically active polypeptide is as shown in SEQ ID NOs: 2-9 (preferably SEQ ID NOs: 2, 6, 7, 9), the optically active polypeptide is located at the 254/256 site of the phosphoenolpyruvate-sensitive polypeptide, and the optical probe has the following mutations: (1) I253D, Q254L, G256V, G257L of the phosphoenolpyruvate-sensitive polypeptide and 1T, 246F of the optically active polypeptide; (2) I253S, Q254L, G256V, G257L of the phosphoenolpyruvate-sensitive polypeptide and 1T, 246F of the optically active polypeptide; (3) I253Q, Q254L, G256V, G257L of the phosphoenolpyruvate-sensitive polypeptide and 1T, 246F of the optically active polypeptide; (4) V54Q, I253Q, Q254L, G256V, G257L of the phosphoenolpyruvate-sensitive polypeptide and 1T, 246F of the optically active polypeptide; (5) N59D, I253Q, Q254L, G256V, G257L of the phosphoenolpyruvate-sensitive polypeptide and 1T, 246F of the optically active polypeptide; (6) R212M, I253Q, Q254L, G256V, G257L of the phosphoenolpyruvate-sensitive polypeptide and 1T, 246F of the optically active polypeptide; (7) G213T, I253Q, Q254L, G256V, G257L of the phosphoenolpyruvate-sensitive polypeptide and 1T, 246F of the optically active polypeptide; (8) K215E, I253Q, Q254L, G256V, G257L of the phosphoenolpyruvate-sensitive polypeptide and 1T, 246F of the optically active polypeptide; (9) L320F, I253Q, Q254L, G256V, G257L of the phosphoenolpyruvate-sensitive polypeptide and 1T, 246F of the optically active polypeptide; (10) S216L, I253Q, Q254L, G256V, G257L of the phosphoenolpyruvate-sensitive polypeptide and 1T, 246F of the optically active polypeptide; (11) S216Y, I253Q, Q254L, G256V, G257L of the phosphoenolpyruvate-sensitive polypeptide and 1T, 246F of the optically active polypeptide.

The present invention further provides a fusion polypeptide, comprising the optical probe described in any of the embodiments herein and other polypeptides. In some embodiments, the other polypeptides are located at the N-terminus and/or C-terminus of the optical probe. In some embodiments, the other polypeptides comprise polypeptides for targeting the optical probe to different organelles or sub - organelles, tags for purification, or tags for immunoblotting.

The present invention also provides a nucleic acid molecule, comprising: (a) the coding sequence of the protein variant, optical probe or fusion polypeptide described in any of the embodiments herein, or (b) the complementary sequence of (a), or (c) a fragment of (a) or (b). The fragment is a primer.

The present invention further relates to variants of the nucleic acid molecules, comprising nucleic acid sequences encoding fragments, analogs, derivatives, soluble fragments and variants of the protein variants, optical probes or fusion polypeptides of the present invention, or complementary sequences thereof.

The present invention further provides a nucleic acid construct comprising the nucleic acid molecule described herein. The nucleic acid sequence encodes the protein variant, optical probe or fusion polypeptide according to any embodiment of the present invention.

In one or more embodiments, the nucleic acid construct is a cloning vector, an expression vector or a recombinant vector.

In one or more embodiments, the nucleic acid molecule is operably linked to an expression control sequence.

In some embodiments, the expression vector is selected from prokaryotic expression vectors, eukaryotic expression vectors and viral vectors.

In another aspect, the present invention further provides a host cell, the host cell: (1) expresses the optical probe or fusion polypeptide according to any embodiment of the present invention; (2) comprises the nucleic acid molecule according to any embodiment of the present invention; or (3) comprises the nucleic acid construct according to any embodiment of the present invention. Preferably, the host cell is Escherichia coli.

In another aspect, the present invention further provides a phosphoenolpyruvate detection kit, comprising the optical probe, fusion polypeptide, or polynucleotide described herein, or an optical probe prepared by the method described herein.

In one or more embodiments, the kit further comprises one or more reagents selected from the group consisting of: buffers, culture media, and phosphoenolpyruvate standards.

In another aspect, the present invention provides a method for preparing the optical probe described herein, comprises: providing a host cell that expresses the optical probe or fusion polypeptide described herein, culturing the host cell under conditions that allow the cell to express, and isolating the optical probe or fusion polypeptide.

In one or more embodiments, the method includes the following steps: 1) incorporating a nucleic acid molecule encoding the optical probe or fusion polypeptide described herein into an expression vector; 2) transferring the expression vector into a host cell; 3) culturing the host cell under conditions suitable for the expression of the expression vector; 4) isolating the optical probe or fusion polypeptide.

In another aspect, the present invention further provides a method for detecting phosphoenolpyruvate in a sample, comprising: contacting the optical probe, fusion polypeptide or host cell described herein with the sample, and detecting the changes in the optically active polypeptide. The detection can be carried out in vivo, in vitro, at the sub-cellular level or in situ. The sample can be, for example, blood.

In another aspect herein, the present invention further provides a method for quantifying phosphoenolpyruvate in a sample, comprising: contacting the optical probe, fusion polypeptide or host cell described herein with the sample, detecting the optical changes of the optically active polypeptide, and quantifying the phosphoenolpyruvate in the sample according to the optical changes of the optically active polypeptide.

In another aspect, the present invention provides a method for screening compounds (e.g., drugs), comprising: contacting the optical probe, fusion polypeptide or host cell described herein with a candidate compound in a system containing phosphoenolpyruvate, detecting the optical changes of the optically active polypeptide, and screening the compounds according to the optical changes of the optically active polypeptide. The method can be used for high-throughput screening of compounds.

In one or more embodiments, the host cell described herein is contacted with a candidate compound in a system containing phosphoenolpyruvate, and the optical changes of the optically active polypeptide indicate whether the candidate compound can regulate the cellular uptake of phosphoenolpyruvate.

In another aspect, the present invention provides a method for intracellular and/or extracellular localization of phosphoenolpyruvate, comprising: contacting a system containing phosphoenolpyruvate with the optical probe or the host cell, and detecting the optical changes of the optically active polypeptide.

In one or more embodiments, the system is a solution, a cellular, or a subcellular system.

In another aspect, the present invention further provides the use of the phosphoenolpyruvate optical probe, fusion polypeptide or host cell described herein in detecting phosphoenolpyruvate in a sample, screening compounds, or intracellular/extracellular localization of phosphoenolpyruvate. In one or more embodiments, the localization is real-time localization.

Beneficial effects of the present invention: the phosphoenolpyruvate optical probe of the present invention is easy to mature, has large dynamic change in fluorescence and excellent specificity, and is able of being expressed in cells by a genetically manipulated method, allows real time in situ, high-throughput, and quantitative detection of phosphoenolpyruvate inside and outside cells, eliminates the time-consuming sample processing steps. Experimental results show that the highest response to phosphoenolpyruvate by the phosphoenolpyruvate optical probe of the present application is more than 5-fold higher than that of the control, and that the localized, qualitative, and quantitative detection can be performed in subcellular structures such as cytosol, mitochondria, nucleus, endoplasmic reticulum, lysosomes, and Golgi apparatus, and high throughput screening of compounds as well as quantitative detection of phosphoenolpyruvate in blood may be performed.

### Description of drawings

The invention will be further described below with reference to the accompanying drawings and examples.
Figure 1 is the SDS-PAGE of an exemplary phosphoenolpyruvate optical probe;
Figure 2 is a diagram of the fluorescence spectral properties of an exemplary phosphoenolpyruvate optical probe;
Figure 3 is a titration curve of an exemplary phosphoenolpyruvate optical probe for phosphoenolpyruvate at different concentrations;
Figure 4 is a summary of the specific detection of an exemplary phosphoenolpyruvate optical probe for the other three substrates, carbohydrate metabolism intermediates and analogues;
Figure 5 is a photograph of the subcellular organelle localization of an exemplary phosphoenolpyruvate optical probe in mammalian cells;
Figure 6 is a schematic diagram of the dynamic monitoring of the concentration of phosphoenolpyruvate in the cytosol by an exemplary phosphoenolpyruvate optical probe in mammalian cells;
Figure 7 is a dot plot of high-throughput compound screening at the living cell level by an exemplary phosphoenolpyruvate optical probe;
Figure 8 is a bar chart of the quantification of phosphoenolpyruvate in the blood of mice and humans by an exemplary phosphoenolpyruvate optical probe.

### DETAILED DESCRIPTION

When a value or range is given, the term "about" as used herein means that the value or range is within 20%, within 10% and within 5% of the given value or range.

The terms "comprise", "include", and equivalent forms thereof include the meaning of "contain" as well as "consist of", for example a composition "comprising" X may consist of X alone or may contain other substances, such as X+Y.

The term "phosphoenolpyruvate-sensitive polypeptide" used herein refers to a polypeptide that responds to phosphoenolpyruvate, and the response includes any response of the chemical, biological, electrical or physiological parameters of the polypeptide that is related to the interaction with the sensitive polypeptide. The responses include minor changes, for example, changes in the orientation of amino acids or peptide fragments of the polypeptide, as well as changes in the primary, secondary, or tertiary structure of the polypeptide, including, for example, changes in protonation, electrochemical potential, and/or conformation. "Conformation" is the three-dimensional arrangement of the primary, secondary, and tertiary structures of a molecule that contains side groups; the conformation changes when the three-dimensional structure of the molecule undergoes a transformation. Examples of conformational changes include the transition from an α-helix to a β-sheet or from a β-sheet to an α-helix. It should be understood that detectable alterations need not be conformational changes as long as the fluorescence of the fluorescent protein moiety is altered. The phosphoenolpyruvate-sensitive polypeptides described herein may also include functional variants thereof. The functional variants of the phosphoenolpyruvate-sensitive polypeptides include, but are not limited to, variants that can interact with phosphoenolpyruvate and thereby undergo the same or similar changes as the parental phosphoenolpyruvate-sensitive polypeptides.

The phosphoenolpyruvate-sensitive polypeptides described in this invention include, but are not limited to, phosphofructokinase TtPFK derived from the extreme thermophilic bacterium Thermus thermophilus or variants having more than 90% homology with it. The phosphoenolpyruvate binding protein can sense the changes in the concentration of phosphoenolpyruvate, and during the dynamic change of the phosphoenolpyruvate concentration, the spatial conformation of the phosphoenolpyruvate binding protein will also change. Truncated variants of TtPFK can also be used in the present invention.

The term "optical probe" used herein refers to a phosphoenolpyruvate-sensitive polypeptide fused to an optically active polypeptide. The inventors discovered that the conformational changes produced by binding a phosphoenolpyruvate-sensitive polypeptide, such as a phosphoenolpyruvate binding protein to a physiological concentration of phosphoenolpyruvate specifically by phosphoenolpyruvate sensitive polypeptides causes conformational changes in an optically active polypeptides, such as fluorescent proteins, which in turn result in the alteration of the optical properties of the optically active polypeptides. A standard curve is plotted with the help of the fluorescence of fluorescent proteins determined at different concentrations of phosphoenolpyruvate allows the presence and/or level of phosphoenolpyruvate to be detected and analyzed. When describing the optical probe of the present invention (for example, when describing the insertion site or the mutation site), the amino acid residue numbering referred to is based on SEQ ID NO: 1.

In the optical probe of the present invention, an optically active polypeptide (such as a fluorescent protein) is operably inserted into a phosphoenolpyruvate-sensitive polypeptide. The "optically active polypeptide" based on protein is a polypeptide capable of emitting fluorescence. Fluorescence is an optical property of an optically active polypeptide, which can be used as a means to detect the responsiveness of the optical probe of the present invention. As used herein, the term "fluorescence property" refers to the molar extinction coefficient at an appropriate excitation wavelength, the fluorescence quantum efficiency, the shape of the excitation spectrum or emission spectrum, the maximum of the excitation wavelength and the maximum of the emission wavelength, the amplitude of excitation at two different wavelengths, the ratio of the emission amplitudes at two different wavelengths, the excited state lifetime, or the fluorescence anisotropy. A measurable difference in any of these properties between the active and inactive states is sufficient for the utility of the fluorescent protein substrate of the present invention in the activity assay. A measurable difference can be determined by quantifying any of the fluorescence properties, for example, the amount of fluorescence at a specific wavelength or the integration of fluorescence over the emission spectrum. Preferably, the protein substrate is selected to have fluorescence characteristics that can be easily distinguished between the unactivated and activated conformational states. The optically active polypeptides described herein may also include functional variants thereof. Functional variants of the optically active polypeptides include, but are not limited to, variants that can exhibit the same or similar changes in fluorescence properties as the parental optically active polypeptide.

The term "fluorescent protein" used herein refers to a protein that fluoresces upon irradiation with excitation light. Fluorescent proteins have been used as basic detection means in the field of biological sciences, such as green fluorescent protein (GFP) commonly used in biotechnology and circularly rearranged blue fluorescent protein (cpBFP), circularly rearranged green fluorescent protein (cpGFP), circularly rearranged yellow fluorescent protein (cpYFP) derived from mutations in this protein, etc; there are also a red fluorescent protein (RFP) commonly used in the art, and circularly rearranged proteins derived from this protein, such as cpmApple, cpmOrange, cpmKate, and others. Exemplarily, cpYFP is as shown in SEQ ID NO: 2, cpmOrange is as shown in SEQ ID NO: 3, cpmKate is as shown in SEQ ID NO: 4 or 8, mCherry is as shown in SEQ ID NO: 5, cpGFP is as shown in SEQ ID NO: 6, cpBFP is as shown in SEQ ID NO: 7, and cpmApple is as shown in SEQ ID NO: 9.

The fluorescent proteins in the optical probe also include functional variants with mutations, including but not limited to fluorescent proteins with mutations at amino acid 1 and/or at amino acid 246 corresponding to SEQ ID NO: 2. The mutation at position 1 is preferably mutated to S, G, N, T, P, A, E or H. The mutation corresponding to position 246 of SEQ ID NO: 2 is preferably mutated to F, C, Wor H. In some embodiments, the functional variant of the fluorescent protein has the sequence shown in any of SEQ ID NO: 2-9 and has mutations selected from any of the following groups at amino acid 1 and amino acid 246 corresponding to SEQ ID NO: 2: 1S and 246F; 1G and 246F; 1H and 246F; 1A and 246C; 1G and 246C; 1S and 246C; 1T and 246C; 1T and 246F. 1S means that the amino acid 1 is mutated to S, and 246F means that the amino acid 246 is mutated to F, and so on.

In the optical probe of the present invention, the optically active polypeptide is located in residues 68-71, 168-173 and/or 253-257 of the phosphoenolpyruvate-sensitive polypeptide in the N-C direction, or replaces the residues therein, and the numbering corresponds to the full length of the phosphoenolpyruvate-sensitive polypeptide. Herein, if the two numbers at the site represented in the form of "X/Y" are consecutive integers, it indicates that the optically active polypeptide is located between the amino acids indicated by the numbers. For example, the insertion site 254/255 indicates that the optically active polypeptide is located between amino acid 254 and amino acid 255 of the phosphoenolpyruvate-sensitive polypeptide. If the two numbers at the site represented in the form of "X/Y" are not consecutive integers, it indicates that the optically active polypeptide replaces the amino acids between the amino acids indicated by the numbers. For example, the insertion site 254/256 indicates that the optically active polypeptide replaces amino acid 255 of the phosphoenolpyruvate-sensitive polypeptide. In an exemplary embodiment, the optically active polypeptide shown in SEQ ID NO: 2, 6, 7 or 9 is located at any one or more of the following sites of the phosphoenolpyruvate-sensitive polypeptide shown in SEQ ID NO: 1: 68/69, 68/70, 69/70, 68/71, 69/71, 70/71, 168/169, 168/170, 169/170, 168/171, 169/171, 170/171, 168/172, 169/172, 170/172, 171/172, 168/173, 169/173, 170/173, 171/173, 172/173, 253/254, 253/255, 254/255, 253/256, 254/256, 255/256, 253/257, 254/257, 255/257 and/or 256/257.

When referring to a certain polypeptide or protein, the term "variant" or "mutant" used in the present invention includes variants that have the same function as the polypeptide or protein but differ in sequence. Variants of the polypeptide or protein may include: homologous sequences, conservative variants, allelic variants, natural mutants, and induced mutants. These variants include, but are not limited to: deletions, insertions, and/or substitutions of one or more (usually 1 - 30, preferably 1 - 20, more preferably 1 - 10, most preferably 1 - 5) amino acids in the sequence of the polypeptide or protein, as well as sequences obtained by adding one or several (usually within 20, preferably within 10, more preferably within 5) amino acids to its carboxy - terminus and/or amino - terminus. These variants may also include polypeptides or proteins with at least about 70%, at least about 75%, at least about 80%, at least about 85%, at least about 90%, at least about 95%, at least about 98%, at least about 99%, or 100% sequence identity to the polypeptide or protein.Not limited by theory, an amino acid residue is altered without changing the overall configuration and function of the polypeptide or protein, i.e., a functionally conserved mutation. For example, in the art, substitution with amino acids of similar or approaching properties generally does not change the function of the polypeptide or protein. In the art, amino acids with similar properties often refer to families of amino acids with similar side chains, which have been well - defined in the art. These families include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with non - polar side chains (e.g., alanine, valine, leucine, isoleucine, arginine, phenylalanine, methionine, tryptophan), amino acids with β - branched side chains (e.g., threonine, valine, isoleucine), and amino acids with aromatic side chains (e.g., tyrosine, phenylalanine, tryptophan, histidine). Another example is that adding one or several amino acids to the amino - terminus and/or carboxy - terminus usually does not change the function of the polypeptide or protein. Conservative amino acid substitutions of many commonly known non - genetically encoded amino acids are known in the art. Conservative substitutions of other non-coding amino acids can be determined based on a comparison of their physical properties with those of genetically encoded amino acids.

A "linker" or "linking region" refers to the amino acid or nucleotide sequence that connects two parts in the polypeptide, protein or nucleic acid of the present invention. Exemplarily, the number of amino acids at the amino terminus of the linking region between the phosphoenolpyruvate-sensitive polypeptide and the optically active polypeptide in the invention is selected to be 0-3, and the number of amino acids at the carboxyl terminus is selected to be 0-2; when a recombinant optical probe is linked to a functional protein as a basic unit, it can be fused at the amino acid or carboxyl terminus of the recombinant optical probe. The linker sequence may be a short peptide chain composed of one or more flexible amino acids, such as Y.

The inventors discovered that variants of the phosphoenolpyruvate-binding protein with mutations at the sites selected from the following exhibit binding activities different from that of phosphoenolpyruvate: I253, Q254, R255, G256, G257, R21, V54, R55, V57, A58, N59, I60, R72, R212, G213, K215, S216, L320 of SEQ ID NO:1. The amino acid mutations include modifications, substitutions or deletions of amino acids.

The present invention provides variants of the phosphoenolpyruvate-binding protein with these mutations, as well as optical probes that comprise such phosphoenolpyruvate-binding protein variants as the phosphoenolpyruvate-sensitive polypeptide. Therefore, in one or more embodiments, the phosphoenolpyruvate-sensitive polypeptide in the optical probe is a phosphoenolpyruvate-binding protein variant as described in any of the embodiments herein, and the fluorescent protein in the optical probe is as shown in SEQ ID NO: 2-9 or a functional variant thereof.

In some specific embodiments, the phosphoenolpyruvate-sensitive polypeptide in the optical probe is as shown in SEQ ID NO:1, the optically active polypeptide is as shown in SEQ ID NO:2, the optically active polypeptide is located at the 254/255 or 254/256 site of the phosphoenolpyruvate-sensitive polypeptide, and the mutations of the optical probe are as shown in any row of Table 5 and Table 6.

In two or more sequences of a polypeptide or nucleic acid molecule, the terms "identity" or "percent identity" refer to when the maximum correspondence is compared and aligned by manual alignment and visual inspection using methods known in the art such as sequence comparison algorithms, in a comparison window or a specified region, two or more sequences or subsequences are identical or have a certain percentage of amino acid residues or nucleotides that are identical in the specified region (e.g., 60%, 65%, 70%, 75%, 80%, 85%, 90%, 91%, 92%, 93%, 94%, 95%, 96%, 97%, 98%, 99%, or 100% identical). For example, the preferred algorithms suitable for determination of percent identity and percent similarity are the BLAST and BLAST 2.0 algorithms, respectively, see Altschul et al., (1977) nucleic acids res. 25:3389 and Altschul et al., (1990) J. mol. Biol 215:403.

Those skilled in the art is well known that in gene cloning operation, it is often necessary to design a suitable restriction enzyme cutting site, which is bound to introduce one or more irrelevant residues at the ends of the expressed polypeptide or protein, and this does not affect the activity of the polypeptide or protein of interest. Also for constructing fusion proteins, facilitating the expression of recombinant proteins, obtaining recombinant proteins that are automatically secreted outside the host cell, or facilitating the purification of recombinant proteins, it is often necessary to add some amino acids to the N-terminus, C-terminus, or other suitable regions within the recombinant protein, for example, including but not limited to, suitable linker peptides, signal peptides, leader peptides, terminal extensions, glutathione S-transferases (GSTs) maltose E-binding protein, Protein A, tags such as 6His or flag, or the proteolytic enzyme sites of Factor Xa or thrombin or enterokinase.

The terms "functional fragment", "derivative" and "analog" used herein refer to proteins that substantially retain the same biological function or activity as the original polypeptide or protein (e.g., a phosphoenolpyruvate-binding protein or a fluorescent protein). The functional variants, derivatives or analogs of the polypeptide or protein of the present invention (e.g., a phosphoenolpyruvate-binding protein or a fluorescent protein) may be (i) a protein in which one or more conservative or non-conservative amino acid residues (preferably conservative amino acid residues) are substituted, and the substituted amino acid residues may or may not be encoded by the genetic code, or (ii) a protein having a substituent group in one or more amino acid residues, or (iii) a protein formed by fusing a mature protein with another compound (such as a compound that prolongs the half - life of the protein, such as polyethylene glycol), or (iv) a protein formed by fusing an additional amino acid sequence to this protein sequence (such as a secretion sequence or a sequence used to purify this protein or a proprotein sequence, or a fusion protein formed with an antigen - IgG fragment). According to the teaching herein, these functional variants, derivatives, and analogues belong to the common knowledge to those skilled in the art. The analogs also include analogs having residues different from the natural L - amino acids (e.g., D - amino acids), and analogs having non - naturally occurring or synthetic amino acids (e.g., β, γ - amino acids). It should be understood that the phosphoenolpyruvate-sensitive polypeptide of the present invention is not limited to the representative proteins, variants, derivatives and analogs listed above. The modified forms (usually without changing the primary structure) include: chemically derived forms of the protein in vivo or in vitro, such as acetylation or carboxylation. Modifications also include glycosylation, such as those proteins produced by glycosylation modifications during the synthesis and processing of the protein or in further processing steps. Such modifications can be accomplished by exposing the protein to enzymes that perform glycosylation (such as mammalian glycosylases or deglycosylases). The modified forms also include sequences having phosphorylated amino acid residues (e.g., phosphotyrosine, phosphoserine, phosphothreonine). Proteins that have been modified to improve their resistance to proteolysis or to optimize the solubility properties are also included.

The present fusion polypeptide comprises the optical probe described herein and an additional polypeptide. In some embodiments, the optical probe described herein also comprises another polypeptide fused to it. The additional polypeptide described herein does not affect the property of the optical probe. The additional polypeptide may be located N- and/or C-terminus to the optical probe. In some embodiments, the additional polypeptide includes polypeptides that localize the optical probe to different organelles or subcellular organelles, a tag for purification, or a tag for immunoblotting. There may be a linker between the optical probe and the additional polypeptide in the fusion polypeptide described herein.

The subcellular organelles described herein include cytosol, mitochondria, nucleus, endoplasmic reticulum, cell membrane, Golgi apparatus, lysosome, and peroxisome, among others. In some embodiments, the tag for purification or the tag for immunoblotting include 6 histidine (6*His), glutathione sulfurtransferase (GST), Flag.

The present invention includes nucleic acid molecules encoding the phosphoenolpyruvate-sensitive polypeptide or the optical probe of the present invention. The terms "nucleic acid" or "nucleotide" or "polynucleotide" or "nucleic acid sequence" used herein may be in the form of DNA or in the form of RNA. DNA form includes cDNA, genomic DNA, or artificially synthesized DNA. DNA can be single stranded or double stranded. DNA can be either the coding or noncoding strand. When referring to nucleic acids, the term "variant" used herein may be a naturally occurring allelic variant or a non-naturally occurring variant. These nucleotide variants include degenerate variants, substitution variants, deletion variants, and insertion variants. As is known in the art, an allelic variant is a form of substitution of a nucleic acid, which may be a substitution, deletion, or insertion of one or more nucleotides but does not substantially alter the function of the protein it encodes. The nucleic acid of the invention may comprise a nucleotide sequence having sequence identity of at least about 50%, at least about 60%, at least about 70%, at least about 75%, at least about 80%, at least about 85%, at least about 90%, at least about 95%, at least about 98%, at least about 99%, or 100% to the nucleic acid sequence. The invention also relates to a nucleic acid fragment that hybridizes to the sequences described above. As used herein, a "nucleic acid fragment" has a length of at least 15 nucleotides, preferably at least 30 nucleotides, more preferably at least 50 nucleotides, and most preferably at least 100 nucleotides or more. The nucleic acid fragment can be used in amplification techniques of nucleic acids (such as PCR).

The full-length sequences or fragments thereof of the optical probes or fusion proteins of the invention can often be obtained by PCR amplification, artificial synthesis or recombinant procedures. Those skilled in the art are known of the procedures of conventional PCR, the synthetic method and the recombinant method, as well as the reagents used. Additionally, mutations can be introduced into the protein sequence of the invention through methods such as mutagenic PCR or chemical synthesis.

The present invention also relates to nucleic acid constructs which contain the polynucleotides described herein and one or more regulatory sequences operably linked to these sequences. The polynucleotides of the present invention can be manipulated in various ways to ensure the expression of the polypeptide or protein. The nucleic acid constructs can be manipulated according to the different requirements of the expression vectors before being inserted into the vectors. Techniques for altering polynucleotide sequences using recombinant DNA methods are known in the art.

In certain embodiments, the nucleic acid construct is a vector. The vector can be a cloning vector, an expression vector, or a homologous recombination vector. The polynucleotides of the present invention can be cloned into many types of vectors, such as plasmids, phagemids, phage derivatives, animal viruses, and cosmids.

A typical expression vector contains expression control sequences that can be used to regulate the expression of the desired nucleic acid sequence, and is operably linked to the nucleic acid sequence described in the present invention or its complementary sequence. The term "expression control sequence" used herein refers to elements that regulate the transcription, translation, and expression of a gene of interest and can be operably linked to the gene of interest, which may be a replication origin, promoter, marker gene, or translational control element, including enhancers, operons, terminators, ribosome binding sites, etc., and the choice of expression control sequence depends on the host cell used. In recombinant expression vectors, "operable ligation" refers to the attachment of the nucleotide sequence of interest to a regulatory sequence in a manner that allows the expression of the nucleotide sequence. Those skilled in the art are well known of methods for constructing expression vectors containing the present fusion protein coding sequences and suitable transcription/translation control signals. These include in vitro recombinant DNA techniques, DNA synthesis techniques, in vivo recombination techniques, etc. The DNA sequence can be effectively ligated to an appropriate promoter in an expression vector to direct mRNA synthesis. Representative examples of these promoters are: the lac or trp promoter of E. coli; λ-phage PL promoter; eukaryotic promoters including the CMV immediate early promoter, the HSV thymidine kinase promoter, the early and late SV40 promoters, the LTR of retroviruses, and several other promoters known to control gene expression in prokaryotic or eukaryotic cells or their viruses. The expression vector also includes a ribosome binding site for translation initiation and a transcription terminator. In one embodiment, the expression vector may use a commercially available pET28a vector with no other special requirements. Exemplarily, BamHI and EcoRI were employed to double digest the nucleotide sequence encoding the optical probe and the expression vector, respectively, and then the enzymatic cleavage products of both were ligated to give recombinant expression vectors. The present invention makes no special limitation to the specific steps and parameters of enzymatic cleavage and ligation, and those conventional in the art would work.

After a recombinant expression vector is obtained, this vector is transformed into host cells to produce a protein or peptide including the fusion protein. This transfer process can be performed with conventional techniques well known to those skilled in the art, such as transformation or transfection. The host cells described herein refer to cells capable of receiving and accommodating recombinant DNA molecules, which are sites of recombinant gene amplification, and ideally the recipient cell should satisfy both conditions of easy access and proliferation. The "host cells" of the invention may include prokaryotic cells and eukaryotic cells, specifically including bacterial cells, yeast cells, insect cells, and mammalian cells. Specific examples can be bacterial cells of E. coli, Streptomyces spp., Salmonella typhimurium, fungal cells such as yeast, plant cells, insect cells of Drosophila S2 or Sf9, CHO, COS, HEK293, HeLa cells, or animal cells such as Bowes melanoma cells, among others, including but not limited to those host cells described above. The host cells are said to prefer a variety of cells favoring the expression or fermentative production of the gene product, such cells being well known and commonly used in the art. Those skilled in the art well know how to select appropriate vectors, promoters, enhancers, and host cells.

The methods of transfer to host cells described herein are conventional methods in the art, including calcium phosphate or calcium chloride coprecipitation, DEAE-mannan-mediated transfection, lipofection, naturally competent cells, chemically mediated transfer, or electroporation. When the host is a prokaryote such as E. coli, the process described is preferably CaCl2 method or MgCl2 method, and their steps used are well known in the art. When the host cell is a eukaryotic cell, the following DNA transfection methods are used: calcium phosphate coprecipitation, conventional mechanical methods such as microinjection, electroporation, liposome packaging, etc.

After transferring the expression vector into host cells, the host cells transferred with the expression vector are subjected to expansion and expression culture, and the phosphoenolpyruvate optical probe is isolated. The host cells are expanded for expression by conventional methods. Depending on the host cell species used, the medium used in culture may be various conventional media. Culture is performed under conditions suitable for host cell growth.

In the present invention, an optical probe is expressed intracellularly, on the cell membrane, or secreted outside the cell. Recombinant proteins can be isolated or purified by various separation methods, if desired, using their physical, chemical, and other properties. The present invention does not have a special limit to a process for separating the phosphoenolpyruvate fluorescent proteins, and a conventional method for the isolation of fusion proteins in the art would work. These methods are well known to those skilled in the art and include, but are not limited to: conventional renaturation treatments, salting out methods, centrifugation, osmotic disruption, sonication, ultra centrifugation, molecular sieving chromatography, adsorption chromatography, ion exchange chromatography, high performance liquid chromatography (HPLC) and other liquid chromatography techniques and the combination of these methods. In one embodiment, His tag affinity chromatography is utilized for optical probe isolation.

The present invention also provides the application of the phosphoenolpyruvate optical probe in real - time localization, quantitative detection of phosphoenolpyruvate and high - throughput compound screening. In one aspect, the phosphoenolpyruvate optical probe is preferably linked to signal peptides of different parts of the cell and then transferred into the cell. The real - time localization of phosphoenolpyruvate can be achieved by detecting the intensity of the fluorescence signal in the cell. The quantitative detection of phosphoenolpyruvate can be carried out by combining the standard titration curve of phosphoenolpyruvate with the change of the fluorescence signal. The change of the fluorescence signal is presented, for example, by the ratio of the standardized fluorescence signals. In the embodiments involving cpYFP, the ratio is the ratio of the ratio of the 485 - nm fluorescence signal to the 420 - nm fluorescence signal of the sample to the corresponding ratio of the control. The standard titration curve of phosphoenolpyruvate according to the present invention is drawn based on the fluorescence signals of the phosphoenolpyruvate optical probe under different concentrations of phosphoenolpyruvate. The phosphoenolpyruvate optical probe of the present invention can be directly transferred into the cell. During the real - time localization and quantitative detection of phosphoenolpyruvate, there is no need for a time - consuming sample processing process, and the results are more accurate. When the phosphoenolpyruvate optical probe of the present invention is used for high - throughput compound screening, different compounds are added to the cell culture medium, and the change in the content of phosphoenolpyruvate is measured to screen out compounds that affect the change in the content of phosphoenolpyruvate. In the present invention, the applications of the phosphoenolpyruvate optical probe in real - time localization, quantitative detection of phosphoenolpyruvate and high - throughput compound screening are all for non-diagnostic and non-therapeutic purposes and do not involve the diagnosis and treatment of diseases.

The present invention also provides a detection kit including the optical probe, nucleic acid molecule, nucleic acid construct and/or cell described herein. The kit also contains other reagents required for the detection of phosphoenolpyruvate. Such other reagents are well known in the art, such as buffers, cell culture media, and phosphoenolpyruvate standards. An exemplary buffer is, for example, 100 mM HEPES and 100 mM NaCl, pH 7.4.

Some specific embodiments
1. A phosphoenolpyruvate binding protein variant:
   (a) having the sequence shown in SEQ ID NO:1 and having mutations at 1, 2, 3, 4, 5 or more sites selected from the group consisting of: I253, Q254, R255, G256, G257, R21, V54, R55, V57, A58, N59, I60, R72, R212, G213, K215, S216, L320, wherein the mutations comprise modifications, substitutions or deletions of amino acids,
   (b) being a sequence having at least 70% sequence identity with (a) and having the mutation described in (a) and retaining the ability to bind to phosphoenolpyruvate,
   preferably, the mutations described in (a) include mutations at sites selected from any one or more of the groups consisting of:
2. An phosphoenolpyruvate optical probe , comprising a phosphoenolpyruvate-sensitive polypeptide and an optically active polypeptide, wherein the optically active polypeptide is located in the sequence of the phosphoenolpyruvate-sensitive polypeptide, and
   the phosphoenolpyruvate-sensitive polypeptide has:
      (1) the sequence of SEQ ID NO: 1, or a sequence having at least 70% sequence identity with it and retaining the binding activity to phosphoenolpyruvate,
      (2) a functional variant of the sequence of SEQ ID NO: 1, which has mutations within 5 amino acids from the junction with the optically active polypeptide, and/or has mutations at 1, 2, 3, 4, 5 or more sites selected from the group consisting of: R21, V54, R55, V57, A58, N59, I60, R72, R212, G213, K215, S216, L320, or
      (3) a sequence having at least 70% sequence identity to the sequence described in (2), having the mutations described in (2), and retaining the sensitivity to phosphoenolpyruvate,
   preferably, (2) is the sequence of the phosphoenolpyruvate-binding protein variant according to embodiment 1.
3. The optical probe as described in embodiment 2, wherein the optically active polypeptide is located in residues 68-71, 168-173 and/or 253-257 of the phosphoenolpyruvate-sensitive polypeptide or substitutes the residues therein.
4. The optical probe as described in embodiment 2, wherein
   the optically active polypeptide is located at any one or more of the following sites of the phosphoenolpyruvate-sensitive polypeptide: 68/69, 68/70, 69/70, 68/71, 69/71, 70/71, 168/169, 168/170, 169/170, 168/171, 169/171, 170/171, 168/172, 169 /172, 170/172, 171/172, 168/173, 169/173, 170/173, 171/173, 172/173, 253/254, 253/255, 254/255, 253/256, 254/256, 255/256, 253/257, 254/257, 255/257 and/or 256/257,
   further preferably,
   the optically active polypeptide is located at any one or more of the following sites of the phosphoenolpyruvate-sensitive polypeptide: 69/71, 170/171, 171/172, 254/255, 254/256 and 255/257,
   further preferably, wherein the optically active polypeptide is a fluorescent protein or a functional variant thereof,
   preferably,
   the fluorescent protein has the sequence shown in any one of SEQ ID NO: 2-9,
   the functional variant of the fluorescent protein has a mutation within 3 amino acids from the junction with the optically active polypeptide; preferably, the functional variant of the fluorescent protein has a mutation at the first amino acid corresponding to SEQ ID NO: 2, and the mutation is preferably selected from Y1S, Y1G, Y1N, Y1T, Y1P, Y1A, Y1E, Y1H,
   the functional variant of the fluorescent protein has a mutation at the amino acid 246 corresponding to SEQ ID NO: 2, and the mutation is preferably selected from N246F, N246C, N246W or N246H,
   further preferably, in the optical probe, the phosphoenolpyruvate-sensitive polypeptide is as shown in SEQ ID NO: 1, and has one or more of the following mutations: I253V, I253N, I253F, I253C, I253E, I253G, I253S, I253K, I253H, I253D, I253P, I253L, I253R, I253T, I253Q, Q254L, Q254V, Q254A, Q254R, Q254P, Q254M, Q254K, Q2541, R255L, R2551, R255F, G256H, G256F, G256Y, G256V, G257L, R21K, V54G, V54K, V54Q, R55S, V57M, A58G, N59D, N59H, N59E, I60W, R72A, R2121, R212E, R212M, G213T, K215H, K215D, K215E, S216D, S216L, S216Y, S2161, S216M, S216F, S216Q, L320Y, L320N and/or L320F, the optically active polypeptide is as shown in SEQ ID NO: 2-9 or is a variant having one or more of the following mutations: Y1S, Y1G, Y1N, Y1T, Y1P, Y1A, Y1E, Y1H, N246F, N246C, N246W, and the optically active polypeptide is located at the 254/255 or 254/256 site of the phosphoenolpyruvate-sensitive polypeptide.
5. A nucleic acid molecule, comprising:
   (a) the coding sequence of the optical probe as described in any one of embodiments 2-4,
   (b) the complementary sequence of (a).
6. A nucleic acid construct comprising the nucleic acid molecule according to embodiment 5,
   preferably, the nucleic acid construct is a cloning vector, an expression vector, or a recombinant vector.
7. A host cell, wherein the host cell:
   (1) expresses the optical probe according to any one of embodiments 2 - 4;
   (2) comprises the nucleic acid molecule according to embodiment 5; or
   (3) comprises the nucleic acid construct according to embodiment 6.
8. A detection kit, comprising:
   (1) the optical probe according to any one of embodiments 2 - 4,
   (2) the nucleic acid sequence according to embodiment 5,
   (3) the nucleic acid construct according to embodiment 6, or
   (4) the host cell according to embodiment 7,
      the detection kit optionally further comprises additional reagents required for detecting phosphoenolpyruvate by the optical probe,
      preferably, the detection kit further comprises one or more reagents selected from the group consisting of: buffer, culture medium, and phosphoenolpyruvate standard.
9. A method for preparing the optical probe according to any one of embodiments 2-4, comprising: providing the host cell according to embodiment 7, culturing the host cell under conditions for the expression of the optical probe, and isolating the optical probe.
10. Use of the optical probe according to any one of embodiments 2-4, the nucleic acid sequence according to embodiment 5, the nucleic acid construct according to embodiment 6, or the host cell according to embodiment 7 in detecting phosphoenolpyruvate in a sample, screening compounds, and localizing phosphoenolpyruvate intracellularly/extracellularly,
   preferably,
   detecting phosphoenolpyruvate in a sample comprises: contacting the optical probe or the host cell with the sample, detecting the optical change of the optically active polypeptide, and detecting phosphoenolpyruvate in the sample according to the optical change of the optically active polypeptide,
   screening compounds comprises: contacting the optical probe or the host cell with a candidate compound in a system comprising phosphoenolpyruvate, detecting the optical change of the optically active polypeptide, and screening the compound according to the optical change of the optically active polypeptide; preferably, screening the compound comprises: contacting the host cell with the candidate compound in a system comprising phosphoenolpyruvate, and the optical change of the optically active polypeptide indicates whether the candidate compound regulates the uptake of phosphoenolpyruvate by cells,
   localizing phosphoenolpyruvate intracellularly/extracellularly comprises: contacting a system comprising phosphoenolpyruvate with the optical probe or the host cell, and detecting the optical change of the optically active polypeptide,
   further preferably, the system is a solution, a cell or a subcellular system.

Herein, concentration, content, percentage and other numerical value can all be expressed in the form of a range. It should also be understood that the use of such a range form is merely for the sake of convenience and conciseness, and it should be flexibly interpreted as including the values explicitly mentioned in the upper and lower limits of the range, and also including all individual numerical values or sub-ranges within that range.

### Examples

The conventional molecular biology cloning methods for genetic engineering and cell culture and imaging methods mainly used in examples are well known to those skilled in the art, for example, Roskams, J., Molecular Biology Laboratory Handbook ; J. Sambrook, D. W. Russell ed, Molecular Cloning: A Laboratory Manual , translated by Peitang Huang et al. (3rd edition, August 2002, Science Press); R. I. Freshney et al., Culture of Animal Cells: a Manual of Basic Technique (5th edition), translated by Jingbo Zhang and Cunquan Xu; Juan S. Bonifacino, M. Daso, et al., Short Protocols in Cell Biology , translated by Jingbo Zhang et al.

The pCDF-cpYFP based, pCDF-phosphoenolpyruvate binding protein plasmid used in examples was constructed by the Protein Laboratory of East China University of Science and Technology, and the pCDF plasmid vector was purchased from Invitrogen. All the primers used for PCR were synthesized, purified, and identified to be correct by mass spectrometry (MS) by Shanghai Generay Biotech Co., Ltd. The expression plasmids constructed in examples were all sequenced by BGI and Jie Li Sequencing. For each example, Taq DNA polymerase was purchased from Dongsheng Bio, pfu DNA polymerase was purchased from Tiangen Biochemical Technology (Beijing) Co., Ltd, primeSTAR DNA polymerase was purchased from Takara, and all three polymerases were purchased with corresponding polymerase buffer and dNTPs. Restriction enzymes such as BamHI, BgIII, HindIII, NdeI, XhoI, EcoRI, SpeI, T4 ligase, and T4 phosphorylase (T4 PNK) were purchased from Fermentas with corresponding buffers and the like. Transfection reagent Lip2000 Kit was purchased from Invitrogen. Compounds such as phosphoenolpyruvate were purchased from Sigma. Chemical reagents such as inorganic salts were purchased from Sigma Aldrich unless specifically stated. HEPES salt, streptomycin sulfate, and puromycin were purchased from Ameresco. 96 well detection black plates, 384 well fluorescence detection black plates were purchased from Grenier.

The DNA purification kit used in examples was purchased from BBI (Canada), and the common Plasmid Mini Preparation Kit was purchased from Tiangen Biochemical Technology (Beijing) Co., Ltd. Clonal strain Mach1 was purchased from Invitrogen. Nickel column affinity chromatography columns and desalting column packing were from GE Healthcare.

The main instruments used in examples included: Biotek Synergy 2 multipurpose microplate reader (Bio-Tek, USA), X-15R high-speed refrigerated centrifuge (Beckman, USA), Microfuge22R tabletop high-speed refrigerated centrifuge (Beckman, USA), PCR amplimer (Biometra, Germany), ultrasonic disruptor (Ningbo SCIENTZ), nucleic acid electrophoresis instrument (Shennengbocai company), spectrofluorometer (Varian, USA), CO2 constant temperature cell culture incubator (SANYO), and inverted fluorescence microscope (Nikon, Japan).

### II. Molecular Biology Methods and Cellular Experimental Methods

### II.1 Polymerase Chain Reaction (PCR):

### 1. PCR Amplification of Target Fragments:

This method was mainly used for gene fragment amplification and colony PCR to identify positive clones. The reaction system for PCR amplification was as follows: template sequence 0.5-1 µL, the forward primer (25 µM) 0.5 µL, the reverse primer (25 µM) 0.5 µL, 10×pfu buffer 5 µL, pfu DNA polymerase 0.5 µL, dNTP (10 mM) 1 µL, sterilized ultrapure water (ddH₂O) 41.5-42 µL, to a total volume of 50 µL. The PCR amplification program was as follows: denaturation at 95 °C for 2-10 min, 30 cycles (94-96 °C for 30-45 s, 50-65 °C for 30-45 s, 72 °C for a period (600 bp/min)), and extension at 72 °C for 10 min.

### 2. PCR Amplification of Long Fragments (>2500 bp):

Long fragment amplification, primarily inverse PCR amplification vector, used in the invention is a technique used to obtain site directed mutations in examples below. Reverse PCR primers were designed at the variation site, where the 5' end of one primer contained the variant nucleotide sequence. Amplified products contained the corresponding mutation site. The reaction system of amplification PCR of long fragments were as follows: template sequence (10 pg-1 ng) 1 µL, the forward primer (25 µM) 0.5 µL, the reverse primer (25 µM) 0.5 µL, 5×PrimerSTAR buffer 10 µL, PrimerSTAR DNA polymerase 0.5 dNTPs (2.5 mM) 4 µL, sterilized ultrapure water (ddH₂O) 33.5 µL, to a total volume of 50 µL. The PCR amplification program was as follows: denaturation at 95 °C for 5 min, 30 cycles (98 °C for 10 s, 50-68 °C for 5-15 s, 72 °C for a period (1000 bp/min)), and extension at 72 °C for 10 min; or denaturation at 95 °C for 5 min, 30 cycles (98 °C for 10 s, 68 °C for a period (1000 bp/min)), and extension at 72 °C for 10 min.

### II.2 Endonuclease Digestion Reaction:

The system for double digestion of plasmid vectors was as follows: plasmid vector 20 µL (approximately 1.5 µg), 10×Buffer 5 µL restriction endonuclease 11-2 µL, restriction endonuclease 21-2 µL, made up to a total volume of 50 µL with sterilized ultrapure water. Reaction conditions 37 °C for 1-7 hours.

### II.3 5' Phosphorylation Reaction of DNA Fragments

Because the plasmid or genomic end extracted from microorganisms contains phosphate groups, but the PCR product does not, the 5' end base of the PCR product needs to be subjected to a phosphate group addition reaction, and only the DNA molecule with phosphate groups on its end allows ligation reaction. The phosphorylation reaction system was as follows: the PCR product fragments DNA sequences 5-8 µL, 10×T4 ligase buffer 1 µL, T4 polynucleotide kinase (T4 PNK) 1 µL, sterilized ultrapure water 0-3 to a total volume of 10 µL. The reaction conditions were 37 °C for 30 min-2 hours followed by inactivation at 72 °C for 20 min.

### II.4 Ligation of Target Fragments and Vectors

There are differences in the ligation method between different fragments and vectors, and three ligation methods are used in this invention.

### 1. Ligation of Blunt End Short Fragments and Linearized Vectors

The principle of this method is that the blunt end product obtained by PCR, after phosphorylation of the 5' end of the DNA fragment by T4 PNK, is ligated with a linearized vector in the presence of PEG4000 and T4 DNA ligase to obtain a recombinant plasmid. The homologous recombination ligation system was as follows: T4 PNK treated DNA fragment 4 µL, linearized vector fragment 4 µL, PEG4000 1 µL, 10×T4 ligase buffer 1 µL, T4 DNA ligase 1 µL, to total of 10 µL. The reaction condition was 22 °C for 30 min.

### 2. Ligation of DNA fragments containing cohesive ends and vector fragments containing cohesive ends

DNA fragments cleaved by restriction enzymes often give rise to overhang sticky ends and can therefore be joined with sticky end vector fragments containing sequence complementarity to form recombinant plasmids. The ligation reaction system was as follows: digested PCR product fragments DNA 1-7 µL, digested plasmid 0.5-7 µL, 10×T4 ligase buffer 1 µL, T4 DNA ligase 1 µL, sterilized ultrapure water added to a total volume of 10 µL. The reaction condition was 16 °C for 4-8 hours.

### 3. Ligation reactions in which the products of DNA fragments phosphorylated at the 5' end were self-circularized after site directed mutagenesis were introduced by inverse PCR

The recombinant plasmid was generated by ligating the 3' and 5' ends of the linearized vector into a DNA fragment phosphorylated at the 5' end by a self-circularization ligation reaction. The reaction system for self-cyclization ligation was as follows: phosphorylation reaction system 10 µL, T4 ligase (5 U/µL) 0.5 µL, to a total volume of 10.5 µL. The reaction condition was 16 °C for 4-16 hours.

### II.5 Preparation and Transformation of Competent Cells

Preparation of Competent Cells:
1. A single colony (e.g., Mach1) is picked and inoculated in 5 ml LB medium at 37 °C, on a shaker overnight.
2. 0.5-1 ml of overnight cultured broth was taken and transferred into 50 ml LB medium and incubated at 37 °C at 220 rpm for 3 to 5 hours until the OD600 reached 0.5.
3. The cells were precooled in ice bath for 2 hours.
4. 4 °C, centrifugation at 4000 rpm for 10 min.
5. The supernatant was discarded, the cells were resuspended with 5 ml of precooled buffer, and resuspension buffer was added again after homogenization to a final volume of 50 ml.
6. Ice bath for 45 min.
7. 4 °C, centrifugation at 4000 rpm for 10 min, the bacteria were resuspended with 5 ml ice precooled storage buffer.
8. 100 µL of broth was loaded to each EP tube and frozen at -80 °C or in liquid nitrogen.

Resuspension buffer: CaCl2 (100 mM), MgCl2 (70 mM), NaAc (40 mM) Storage buffer: 0.5 mL DMSO, 1.9 mL 80% glycol, 1 mL 10×CaCl2 (1 M), 1 mL 10×MgCl2 (700 mM), 1 mL 10×NaAc (400 mM), 4.6 mL ddH₂O

Transformation of Component Cells:
1. Take 100 µL competent cells were taken and thawed on an ice bath.
2. The appropriate volume of ligation product was added, mixed by gently pipetting, and incubated in ice bath for 30 min. The volume of ligation product normally added was less than 1/10 of the volume of competent cells.
3. The broth was put into a 42 °C water bath for heat shock for 90 s and quickly transferred to an ice bath for 5 min.
4. 500 µL LB was added and incubated at 200 rpm on a constant temperature shaker at 37 °C for 1 hour.
5. The broth was centrifuged at 4000 rpm for 3 min and 200 µL supernatant was added to mix the bacteria well and spread evenly on the surface of the agar plate containing appropriate antibiotics, and the plate is inverted in a 37 °C incubator overnight.

### II.6 Expression, Purification, and Fluorescent Detection of Protein

1. The expression vectors (for example, a phosphoenolpyruvate optical probe expression vector based on pCDF.) were transformed into BL21 (DE3) cells, incubated inverted overnight, and the clones were picked from the plates into 250 mL Erlenmeyer flasks, placed in a shaker at 37 °C and incubated at 220 rpm to OD=0.4-0.8, then 1/1000 (V/V) of IPTG (1 M) was added to induce expression at 18 °C for 24-36 hours.
2. Upon completion of inducible expression, cells were harvested by centrifugation at 4000 rpm for 30 min, resuspended in 50 mM phosphate buffer and disrupted ultrasonically until the broth were clear, centrifuged at 9600 rpm at 4 °C for 20 min.
3. The centrifuged supernatant was used to obtain protein by purification through a self-packed nickel column affinity chromatography column, and the protein obtained after nickel column affinity chromatography was further used to obtain protein dissolved in 100 mM HEPES buffer (pH 7.4).
4. After purified proteins were subjected to SDS-PAGE, probes were diluted to a protein solution having a final concentration of 0.2-5 µM using assay buffer (100 mm HEPES, 100 mM NaCl, pH 7.4). Phosphoenolpyruvate was formulated as a stock solution at a final concentration of 50 mM with the assay buffer (100 mM HEPES, 100 mM NaCl, pH 7.4).
5. 100 µl 1 µM of protein solution was taken, incubated at 37 °C for 10 min, phosphoenolpyruvate was added for titration, and the fluorescence intensity of 528 nm emission after 420 nm and 485 nm fluorescence excitation of the protein was measured. The fluorescence excitation and emission determination of the samples were done using a multifunctional fluorescence microplate reader.
6. 100 µl 1 µM of protein solution was taken, incubated at 37 °C for 10 min, and phosphoenolpyruvate was added to determine the absorption and fluorescence spectra of the proteins. Determination of the absorption and fluorescence spectra of the samples was done by spectrophotometer and spectrofluorometer.

### II.7 Transfection of Mammalian Cells and Detection of Fluorescence

1. pCDNA3.1+-based phosphoenolpyruvate optical probe plasmids were transfected into HEK293 by using the transfection reagent Lipofectamine2000 (Invitrogen) and incubated in a cell incubator at 37 °C with 5% CO2. After the exogenous gene was fully expressed for 24~36 hours, the fluorescence detection was performed.
2. After induction of expression, adherent HEK293 cells were washed three times with PBS and placed in HBSS solution for fluorescence microscopy and microplate reader assays, respectively.

### Example 1: Phosphoenolpyruvate Binding Protein Plasmid

The TtPFK gene in the hyperthermophilic archaeon was amplified by PCR and digested with BamHI and EcoRI after gel electrophoresis of the PCR products, while the corresponding double digestion of the pCDF vector was performed. After ligation with T4 DNA ligase, DH5α was transformed with the product and the transformed DH5α was spread onto LB plates (100 ug/ml streptomycin) and placed in culture at 37°C overnight. After growing DH5α transformants were subjected to plasmid extraction, they were subjected to PCR. Positive plasmids were sequenced to be correct for subsequent plasmid construction.

### Example 2: Expression and Detection of Optical Probes with cpYFP Inserted at Different Sites

In this example, the insertion of cpYFP at the following sites was selected according to the crystal structure of the phosphoenolpyruvate binding protein based on pCDF-TtPFK to obtain corresponding pCDF-TtPFK-cpYFP plasmid: 68/69,68/70, 69/70, 68/71, 69/71, 70/71, 168/169, 168/170, 169/170, 168/171, 169/171, 170/171, 168/172, 169/172, 170/172, 171/172, 168/173, 169/173, 170/173, 171/173, 172/173, 253/254, 253/255, 254/255, 253/256, 254/256, 255/256, 253/257, 254/257, 255/257 and 256/257. As an exemplary demonstration only, the amino acid sequence and nucleic acid sequence of 254/255-TtPFK-cpYFP are shown in SEQ ID NO:10 and SEQ ID NO:11 respectively.

DNA fragments of cpYFP were generated by PCR, and the homologous sequences at the terminal of cpYFP were introduced through the 5'-ends of the primers, meanwhile, the linearized pCDF-phosphoenolpyruvate binding protein vector was amplified by PCR, with its 5' and 3' terminals respectively carrying sequences (15 bp - 20 bp) that are completely identical to those of the two terminals of cpYFP. The linearized pCDF-TtPFK and cpYFP fragments undergo homologous recombination under the action of Hieff Clone Enzyme. DH5α was transformed with the product and the transformed DH5α was spread onto LB plates (100 ug/ml streptomycin) and placed in culture at 37°C overnight. The positive clones identified by PCR were subjected to plasmid extraction and subsequent sequencing. The sequencing was completed by Shanghai Jieli Biology Co., Ltd.

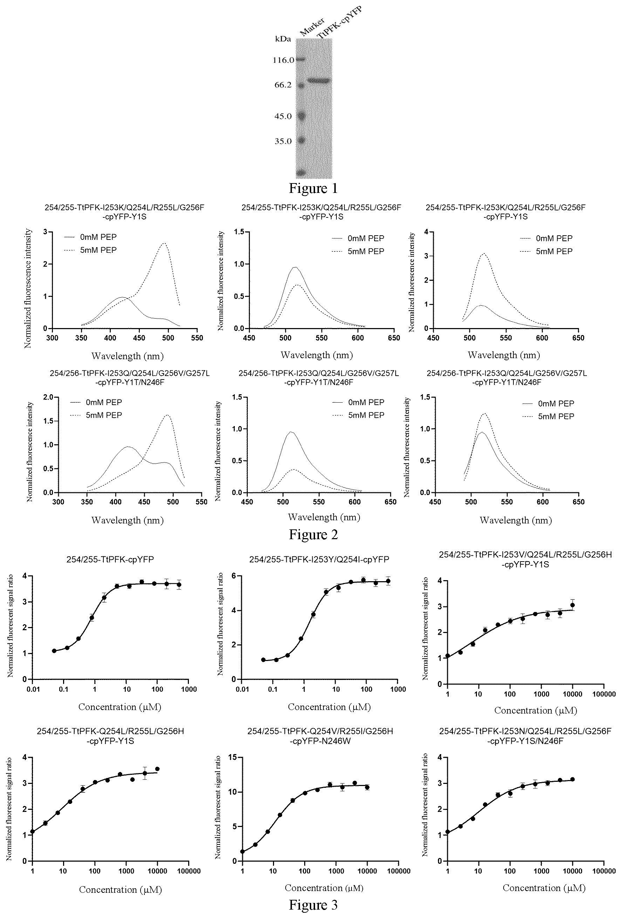

After sequenced to be correct, the recombinant plasmids were transformed into BL21 (DE3) to induce expression, and proteins were purified, electrophoretically sized around 63 kDa by SDS-PAGE. This size was in accordance with the size of the His-tag purification tag containing TtPFK-cpYFP fusion protein. The results were shown in Figure 1.

The disrupted supernatant of Escherichia coli expressing the TtPFK-cpYFP fusion protein was subjected to phosphoenolpyruvate-responsive screening, dividing the detection signal of the fusion fluorescent protein containing 1 mM phosphoenolpyruvate by that of the fusion fluorescent protein without phosphoenolpyruvate. As shown in Table 1, the detection results showed that the optical probes with insertions at sites 69/71, 170/171, 171/172, 254/255, 254/256 and 255/257 in the disrupted supernatant expressing the TtPFK-cpYFP fusion protein responded more than 1.3-fold to phosphoenolpyruvate, wherein the sites 254/255 and 254/256 exhibited responses to phosphoenolpyruvate that were unaffected by other TtPFK-binding substrates.

**Table 1**

| TtPFK | |
|---|---|
| Insertion Site | Normalized fluorescent signal ratio |
| 68/69 | 0.94 |
| 68/70 | 0.99 |
| 69/70 | 0.91 |
| 68/71 | 1.06 |
| 69/71 | 0.77 |
| 70/71 | 1.05 |
| 168/169 | 1.25 |
| 168/170 | 0.99 |
| 169/170 | 1.12 |
| 168/171 | 1.15 |
| 169/171 | 1.03 |
| 170/171 | 1.52 |
| 168/172 | 0.84 |
| 169/172 | 1.02 |
| 170/172 | 1.06 |
| 171/172 | 1.53 |
| 168/173 | 1.00 |
| 169/173 | 0.99 |
| 170/173 | 1.01 |
| 171/173 | 0.98 |
| 172/173 | 1.02 |
| 253/254 | 0.81 |
| 253/255 | 0.99 |
| 254/255 | 2.12 |
| 253/256 | 0.93 |
| 254/256 | 1.90 |
| 255/256 | 1.27 |
| 253/257 | 0.88 |
| 254/257 | 1.03 |
| 255/257 | 1.33 |
| 256/257 | 1.00 |

### Example 3: Expression and Detection of cpGFP Optical Probes with Different Insertion Sites

The phosphoenolpyruvate green fluorescent protein fluorescent probe was constructed by replacing cpYFP with cpGFP as in Example 2. As shown in Table 2, the detection results showed that the optical probes with insertions at sites 171/172, 254/255 and 254/256 or corresponding amino acid sites of their family proteins in the disrupted supernatant expressing the TtPFK-cpYFP fusion protein responded more than 1.3-fold to phosphoenolpyruvate, wherein the sites 254/255 and 254/256 exhibited responses to phosphoenolpyruvate that were unaffected by other TtPFK-binding substrates.

**Table 2**

| TtPFK | |
|---|---|
| Insertion Site | Normalized fluorescent signal ratio |
| 68/69 | 0.91 |
| 68/70 | 0.95 |
| 69/70 | 0.90 |
| 68171 | 1.01 |
| 69/71 | 0.91 |
| 70/71 | 0.98 |
| 168/169 | 1.02 |
| 168/170 | 1.01 |
| 169/170 | 0.91 |
| 168/171 | 0.95 |
| 169/171 | 1.03 |
| 170/171 | 1.28 |
| 168/172 | 1.00 |
| 169/172 | 1.01 |
| 170/172 | 0.92 |
| 171/172 | 1.30 |
| 168/173 | 1.00 |
| 169/173 | 1.08 |
| 170/173 | 1.00 |
| 171/173 | 0.94 |
| 172/173 | 1.08 |
| 253/254 | 0.96 |
| 253/255 | 0.99 |
| 254/255 | 1.57 |
| 253/256 | 1.09 |
| 254/256 | 1.49 |
| 255/256 | 1.04 |
| 253/257 | 0.98 |
| 254/257 | 1.00 |
| 255/257 | 1.23 |
| 256/257 | 0.99 |

### Example 4: Expression and Detection of cpBFP Optical Probes with Different Insertion Sites

The phosphoenolpyruvate blue fluorescent protein fluorescent probe was constructed by replacing cpYFP with cpBFP as in Example 2. As shown in Table 3, the detection results showed that the optical probes with insertions at sites 170/171, 171/172, 254/255 and 254/256 or corresponding amino acid sites of their family proteins in the disrupted supernatant expressing the TtPFK-cpYFP fusion protein responded more than 1.3-fold to phosphoenolpyruvate, wherein the sites 254/255 and 254/256 exhibited responses to phosphoenolpyruvate that were unaffected by other TtPFK-binding substrates.

**Table 3**

| TtPFK | |
|---|---|
| Insertion Site | Normalized fluorescent signal ratio |
| 68/69 | 0.87 |
| 68/70 | 1.07 |
| 69/70 | 1.08 |
| 68/71 | 0.98 |
| 69/71 | 0.94 |
| 70/71 | 1.01 |
| 168/169 | 1.04 |
| 168/170 | 0.95 |
| 169/170 | 0.88 |
| 168/171 | 0.99 |
| 169/171 | 1.02 |
| 170/171 | 1.42 |
| 168/172 | 1.04 |
| 169/172 | 0.99 |
| 170/172 | 1.04 |
| 171/172 | 1.45 |
| 168/173 | 1.06 |
| 169/173 | 1.08 |
| 170/173 | 0.88 |
| 171/173 | 1.03 |
| 172/173 | 0.95 |
| 253/254 | 1.08 |
| 253/255 | 1.02 |
| 254/255 | 1.61 |
| 253/256 | 0.85 |
| 254/256 | 1.35 |
| 255/256 | 1.04 |
| 253/257 | 0.95 |
| 254/257 | 1.05 |
| 255/257 | 1.24 |
| 256/257 | 1.11 |

### Example 5: Expression and Detection of cpmApple Optical Probes with Different Insertion Sites

The phosphoenolpyruvate red fluorescent protein fluorescent probe was constructed by replacing cpYFP with cpmApple as in Example 2. As shown in Table 4, the detection results showed that the optical probes with insertions at sites 170/171, 171/172, 254/255 and 254/256 or corresponding amino acid sites of their family proteins in the disrupted supernatant expressing the TtPFK-cpYFP fusion protein responded more than 1.3-fold to phosphoenolpyruvate, wherein the sites 254/255 and 254/256 exhibited responses to phosphoenolpyruvate that were unaffected by other TtPFK-binding substrates.

**Table 4**

| TtPFK | |
|---|---|
| Insertion Site | Normalized fluorescent signal ratio |
| 68/69 | 1.05 |
| 68/70 | 0.91 |
| 69/70 | 0.99 |
| 68/71 | 1.07 |
| 69/71 | 0.94 |
| 70/71 | 0.98 |
| 168/169 | 1.04 |
| 168/170 | 0.96 |
| 169/170 | 1.04 |
| 168/171 | 1.07 |
| 169/171 | 0.99 |
| 170/171 | 1.38 |
| 168/172 | 1.00 |
| 169/172 | 0.93 |
| 170/172 | 1.00 |
| 171/172 | 1.30 |
| 168/173 | 0.97 |
| 169/173 | 1.07 |
| 170/173 | 0.98 |
| 171/173 | 1.05 |
| 172/173 | 1.08 |
| 253/254 | 0.95 |
| 253/255 | 1.08 |
| 254/255 | 1.72 |
| 253/256 | 1.00 |
| 254/256 | 1.67 |
| 255/256 | 1.07 |
| 253/257 | 1.05 |
| 254/257 | 0.97 |
| 255/257 | 1.07 |
| 256/257 | 0.94 |

### Example 6: Expression and Detection of cpYFP Optical Probe with Linker Mutation

For the optical probes obtained in Example 2 that respond to phosphoenolpyruvate more than 1.3-fold and are unaffected by other binding substrates of TtPFK, that is, the two optical probes inserted at the 254/255 and 254/256 sites of TtPFK are linearized by inverse PCR, the sequence of the linker mutation site is introduced into the primer, and the obtained PCR products are homologously recombined under the action of Hieff Clone Enzyme to establish a mutation library. Merely for exemplary demonstration, the amino acid sequence and nucleic acid sequence of 254/255-TtPFK-1253K1Q254L1R255L1G256F-cpYFP-Y1S are shown in SEQ ID NO: 12 and 13 respectively.

The recombinant plasmids of the mutation library were transformed into BL21 (DE3) to induce expression, and the purified probe protein was subjected to phosphoenolpyruvate-responsive screening, dividing the detection signal of the fusion fluorescent protein containing 2 mM phosphoenolpyruvate by that of the fusion fluorescent protein without phosphoenolpyruvate. The detection results showed that the optical probes with a response to phosphoenolpyruvate exceeding or equal to 2-fold are shown in Table 5.

**Table 5**

| Insertion Sites | TtPFK | cpYFP mutation | R485/420 | Kd/µM |
|---|---|---|---|---|
| 254/255 | | | 3.66 | 0.8 |
| 254/255 | 1253Y/Q2541 | | 5.32 | 1.55 |
| 254/255 | 1253V/Q254L/R255L/G256H | Y1S | 3.06 | 5.64 |
| 254/255 | Q254L/R255L/G256H | Y1S | 3.55 | 8.5 |
| 254/255 | Q254V/R2551/G256H | N246W | 10.65 | 11.42 |
| 254/255 | 1253N/Q254L/R255L/G256F | Y1S/N246F | 3.16 | 9.66 |
| 254/255 | 1253F/Q254L/R255L/G256F | Y1G/N246F | 5.98 | 5.56 |
| 254/255 | 1253V/Q254A/R255F/G256Y | N246F | 5.27 | 31.99 |
| 254/255 | 1253V/Q254L/R255F/G256Y | Y1H/N246F | 5.85 | 81.67 |
| 254/255 | 1253C/Q254L/R255L/G256F | Y1N | 6.53 | 31.05 |
| 254/255 | I253E/Q254L/R255L/G256F | Y1S | 6.42 | 68.4 |
| 254/255 | 1253G/Q254L/R255L/G256F | Y1T | 4.9 | 16.55 |
| 254/255 | 12531/Q254L/R255L/G256F | Y1S | 3.41 | 45 |
| 254/255 | 1253S/Q254L/R255L/G256F | Y1S | 3.84 | 29.51 |
| 254/255 | 1253G/Q254L/R255L/G256F | Y1P | 8.45 | 30.9 |
| 254/255 | 1253K/Q254L/R255L/G256F | Y1S | 8.03 | 17.52 |
| 254/255 | 1253H/Q254L/R255L/G256F | Y1N | 7.08 | 21.83 |
| 254/255 | 1253D/Q254L/R255L/G256F | Y1S | 5.51 | 44.17 |
| 254/255 | Q254L/R255L/G256F | Y1S | 4.76 | 40.32 |
| 254/255 | 1253P/Q254L/R255L/G256F | Y1T | 3.68 | 6.54 |
| 254/255 | Q254L/R255L/G256F | Y1T | 8.03 | 20.43 |
| 254/255 | 1253L/Q254R/R255L/G256F | | 5.16 | 435.8 |
| 254/255 | 1253R/Q254L/R255L/G256F | Y1S | 6.96 | 18.16 |
| 254/255 | 1253T/Q254L/R255F/G256Y | Y1T | 4.23 | 16.69 |
| 254/255 | 1253Q/Q254P/R255F/G256Y | | 5.66 | 52.13 |
| 254/255 | 1253Q/Q254M/R255F/G256Y | Y1P | 4.83 | 42.67 |
| 254/255 | 1253T/Q254P/R255F/G256Y | Y1A | 2.57 | 12.92 |
| 254/255 | Q254L/R255F/G256F | Y1N | 5.89 | 60.83 |
| 254/255 | 1253S/Q254K/R255F/G256F | Y1E | 3.02 | 32.61 |
| 254/255 | Q254P/R255F/G256F | N246C | 7.42 | 80.64 |
| 254/255 | 1253E/Q254V/R255F/G256F | Y1A/N246C | 6.55 | 74.29 |
| 254/255 | Q254L/R255F/G256F | Y1G/N246C | 3.37 | 5.96 |
| 254/255 | 1253T/Q254L/R255F/G256F | Y1S/N246C | 5.26 | 20.22 |
| 254/255 | Q254L/R255F/G256F | Y1T/N246C | 7.57 | 9.15 |
| 254/256 | | | 1.68 | 2.145 |
| 254/256 | 1253D/Q254L/G256V/G257L | Y1T/N246F | 11.39 | 13.38 |
| 254/256 | 1253S/Q254L/G256V/G257L | Y1T/N246F | 8.83 | 8.93 |
| 254/256 | 1253Q/Q254L/G256V/G257L | Y1T/N246F | 14.02 | 13.08 |

### Example 7: Expression and Detection of cpYFP Optical Probe with Binding Pocket Mutation

For the 254/255-TtPFK-cpYFP obtained in Example 2 and the 254/255-TtPFK-I253G/Q254L/R255L/G256F-cpYFP-Y1P, 254/255-TtPFK-I253K/Q254L/R255L/G256F-cpYFP-Y1S and 254/256-TtPFK-I253Q/Q254L/G256V/G257L-cpYFP-Y1T/N246F optical probes obtained in Example 6, amino acid were mutated at positions R21, R25, V54, R55, V57, A58, N59, I60, I61, R155, G186, R212, G213, K214, K215, S216 and L320 in the phosphoenolpyruvate binding pocket, and at positions R72, R163, R172 and R246 in the ATP binding pocket to adjust the affinity of the phosphoenolpyruvate optical probe to phosphoenolpyruvate and the substrate specificity. The probes were linearized by reverse PCR, and the sequences of the mutation sites were introduced into the primers. The obtained PCR products were subjected to homologous recombination to obtain plasmids containing the above site mutations. The mutant plasmids were transformed into BL21 (DE3) for induced expression, and the mutant probe proteins were purified for the phosphoenolpyruvate response test. The detection signal of the fusion fluorescent protein containing 2 mM phosphoenolpyruvate was divided by the detection signal of the fusion fluorescent protein without phosphoenolpyruvate. The detection results showed that the optical probes with a response to phosphoenolpyruvate exceeding or equal to 2-fold are shown in Table 6.

**Table 6**

| Insertio n Sites | TtPFK mutation | cpYFP mutation | R485/42 0 | Kd/µM |
|---|---|---|---|---|
| 254/255 | R21K | | 2.69 | 715.7 |
| 254/255 | V54K | | 4.49 | 7.84 |
| 254/255 | V54Q | | 4.6 | 23.32 |
| 254/255 | V54G | | 4.42 | 91.13 |
| 254/255 | R55S | | 2.91 | 294.1 |
| 254/255 | V57M | | 3.76 | 0.449 2 |
| 254/255 | A58G | | 3.63 | 0.36 |
| 254/255 | I60W | | 2.57 | 0.495 7 |
| 254/255 | N59D | | 3.8 | 37.43 |
| 254/255 | N59H | | 4.2 | 4.62 |
| 254/255 | N59E | | 4.33 | 177.7 |
| 254/255 | R2121 | | 3.88 | 59.81 |
| 254/255 | R212E | | 3.91 | 103.2 |
| 254/255 | R212M | | 4.16 | 56.47 |
| 254/255 | G213T | | 4.44 | 16.24 |
| 254/255 | K215E | | 3.84 | 29.71 |
| 254/255 | K215H | | 3.82 | 14.28 |
| 254/255 | K215D | | 3.82 | 75.89 |
| 254/255 | S216D | | 4.27 | 202.9 |
| 254/255 | S216L | | 3.03 | 0.254 4 |
| 254/255 | S216Y | | 3.59 | 0.285 3 |
| 254/255 | S2161 | | 3.89 | 0.24 |
| 254/255 | S216M | | 3.75 | 0.25 |
| 254/255 | S216F | | 1.77 | 0.159 8 |
| 254/255 | S216Q | | 3.62 | 0.617 2 |
| 254/255 | L320Y | | 3.33 | 127.3 |
| 254/255 | L320N | | 2.36 | 200.4 |
| 254/255 | L320F | | 3.97 | 70.17 |
| 254/255 | R72A | | 2.72 | 10.26 |
| 254/255 | V54G/R72A | | 3 | 788.8 |
| 254/255 | V54K/I253G/Q254L/R255L/G256F | Y1P | 6.68 | 267.3 |
| 254/255 | V54Q/I253G/Q254L/R255L/G256F | Y1P | 7.24 | 769.5 |
| 254/255 | N59H/I253G/Q254L/R255L/G256F | Y1P | 6.5 | 178.3 |
| 254/255 | V54K/I253K/Q254L/R255L/G256F | Y1S | 8.04 | 129.4 |
| 254/255 | V54Q/I253K/Q254L/R255L/G256F | Y1S | 7.62 | 327.5 |
| 254/255 | N59H/I253K/Q254L/R255L/G256F | Y1S | 7.45 | 87.67 |
| 254/256 | V54Q/I253Q/Q254L/G256V/G257L | Y1T/N246 F | 12.23 | 281.3 |
| 254/256 | N59D/I253Q/Q254L/G256V/G257L | Y1T/N246 F | 7 | 374 |
| 254/256 | R212M/I253Q/Q254L/G256V/G257 L | Y1T/N246 F | 10 | 714.2 |
| 254/256 | G213T/I253Q/Q254L/G256V/G257 L | Y1T/N246 F | 5.73 | 199.3 |
| 254/256 | K215E/I253Q/Q254L/G256V/G257 L | Y1T/N246 F | 7.55 | 249 |
| 254/256 | L320F/I253Q/Q254L/G256V/G257L | Y1T/N246 F | 6.19 | 1773 |
| 254/256 | S216L/I253Q/Q254L/G256V/G257L | Y1T/N246 F | 6.94 | 1.393 |
| 254/256 | S216Y/I253Q/Q254L/G256V/G257 L | Y1T/N246 F | 8.25 | 2.365 |

### Example 8: Performance of the optical probe mutant

Exemplarily, detection of fluorescence spectra was performed using a fluorescence spectrophotometer after subjecting two purified phosphoenolpyruvate optical probes to 0 mM and 5 mM phosphoenolpyruvate for 10 min, respectively.

Determination of excitation spectrum: the excitation spectrum was recorded with an excitation range from 370 nm to 510 nm and an emission wavelength of 530 nm, with readings taken every 5 nm. The results showed that the probe had two excitation peaks at approximately 410 nm and 490 nm, as shown in Figure 2.

Determination of emission spectra: the excitation wavelengths were fixed at 420 nm and 460 nm, and the emission spectra at 470-600 nm and 490-600 nm were recorded, with readings taken every 5 nm. The results are shown in Figure 2.

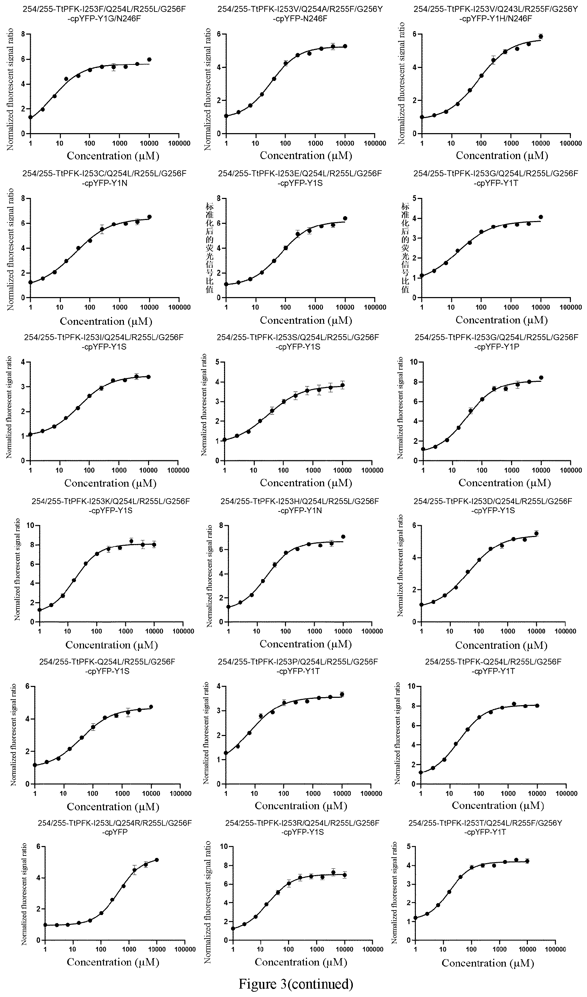

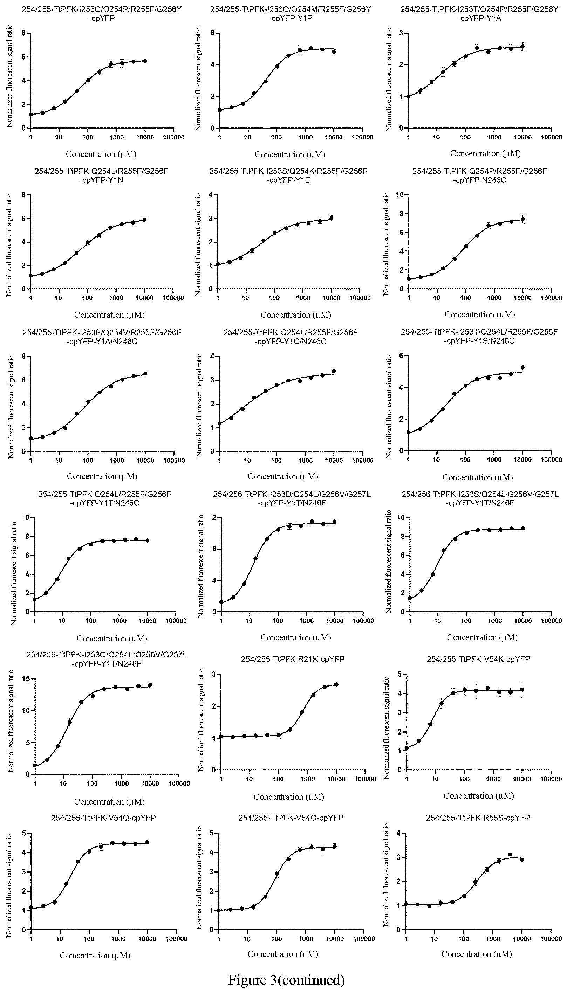

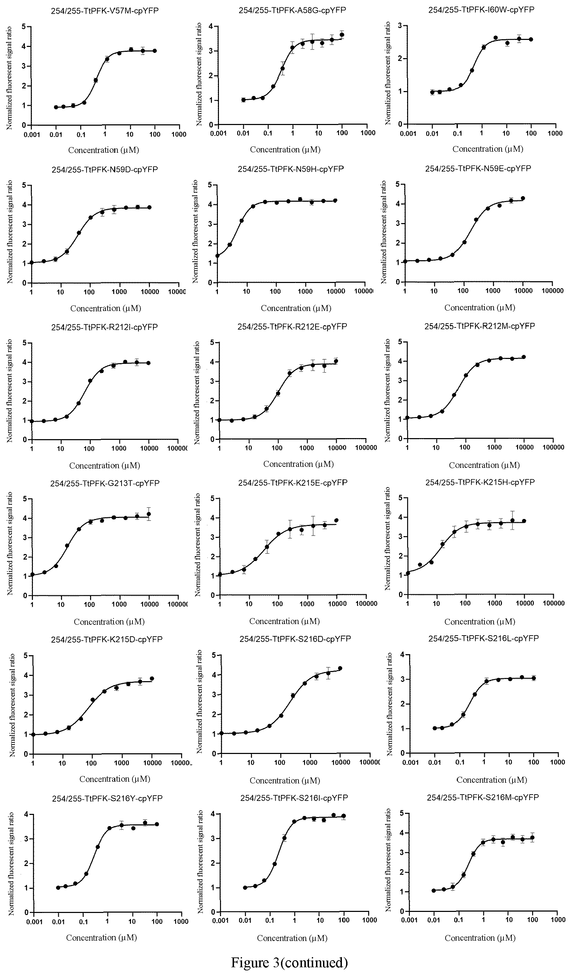

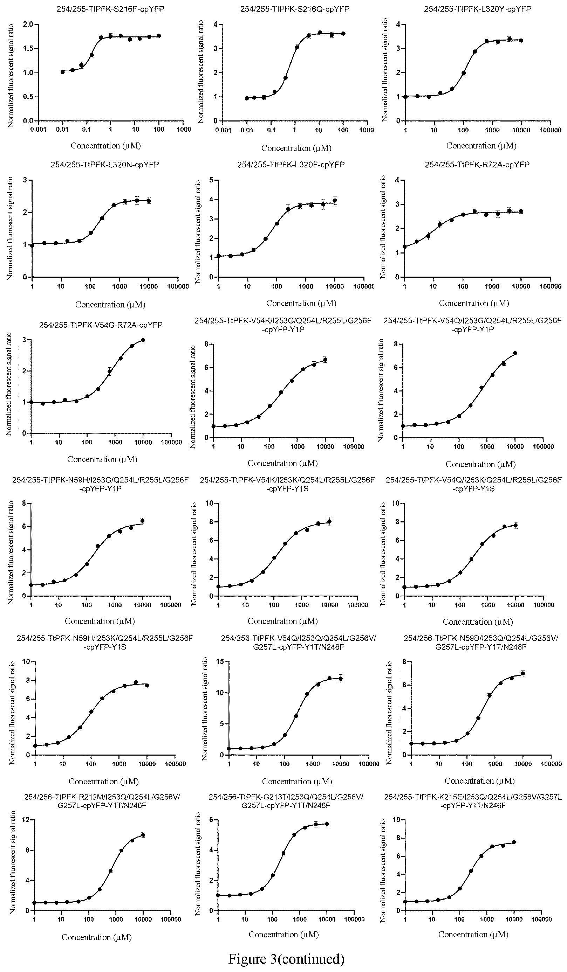

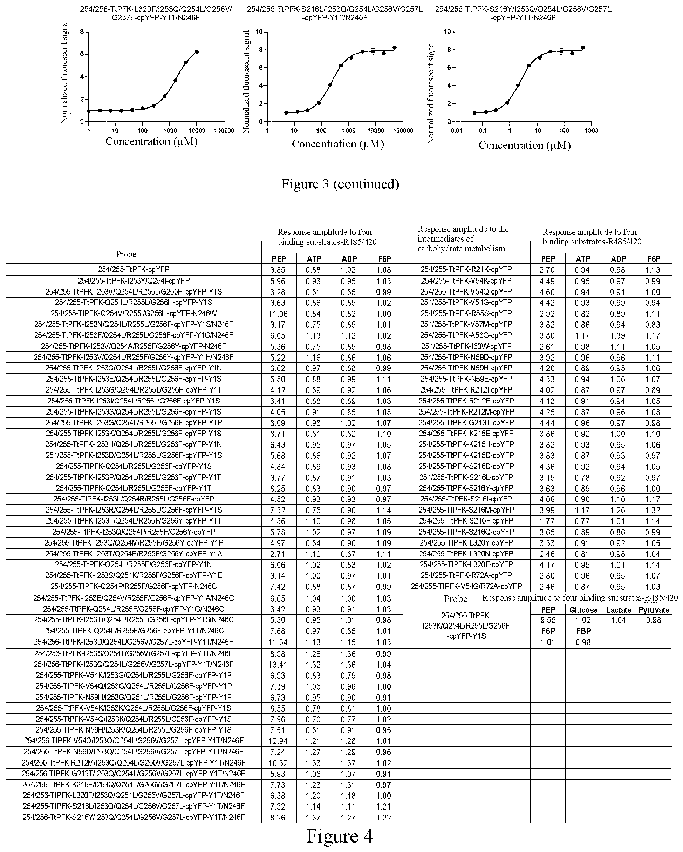

The 81 purified phosphoenolpyruvate probes described in Example 6 and Example 7 were used to detect phosphoenolpyruvate with a concentration gradient ranging from 0 to 10 mM. After treating the purified probes for 10 minutes, the change in the ratio of the fluorescence intensity at an excitation of 420 nm and an emission of 528 nm to that at an excitation of 485 nm and an emission of 528 nm was detected. The results are shown in Figure 3, and the Kd (binding constant) values of the 81 phosphoenolpyruvate optical probes are 0.8, 1.55, 5.64, 8.5, 11.42, 9.66, 5.56, 31.99, 81.67, 31.05, 68.4, 16.55, 45, 29.51, 30.9, 17.52, 21.83, 44.17, 40.32, 6.54, 20.43, 435.8, 18.16, 16.69, 52.13, 42.67, 12.92, 60.83, 32.61, 80.64, 74.29, 5.96, 20.22, 9.15, 13.38, 8.93, 13.08, 715.7, 7.84, 23.32, 91.13, 294.1, 0.4492, 0.36, 0.4957, 37.43, 4.62, 177.7, 59.81, 103.2, 56.47, 16.24, 29.71, 14.28, 75.89, 202.9, 0.2544, 0.2853, 0.24, 0.25, 0.1598, 0.6172, 127.3, 200.4, 70.17, 10.26, 788.8, 267.3, 769.5, 178.3, 129.4, 327.5, 87.67, 281.3, 374, 714.2, 199.3, 249, 1773, 1.393 and 2.365 µM respectively.

The reactivity of the 81 phosphoenolpyruvate optical probes was tested against the three TtPFK-binding substrates ATP, ADP, and F6P, meanwhile, the reactivity of one phosphoenolpyruvate optical probe was tested against carbohydrate metabolism intermediates such as glucose, lactate, pyruvate, F6P, and FBP. The results showed that it had excellent specificity, as shown in Figure 4.

### Example 9: Subcellular Localization of the Optical Probe and the Performance of the Optical Probe in Subcellular Organelles

In this example, different localization signal peptides were used to fuse to the optical probe 254/255-TtPFK- I253K/Q254L/R255L/G256F-cpYFP-Y1S which was localized in different organelles.

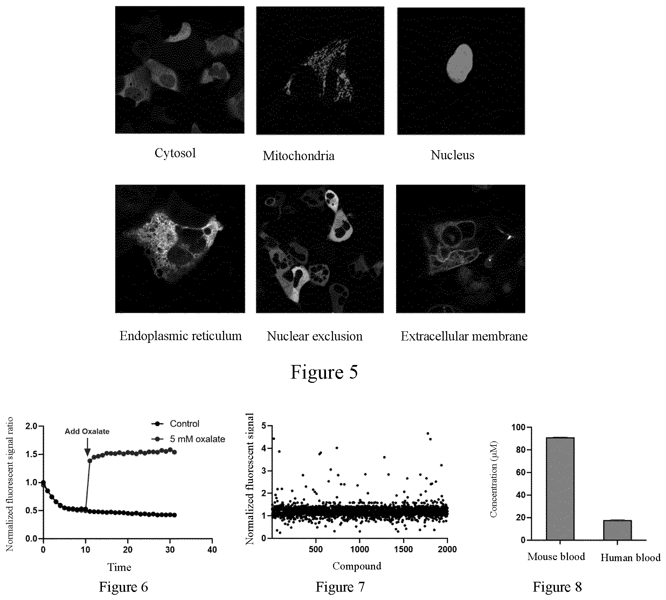

Thirty-six hours after transfection of 293 cells with optical probe plasmids fused to different localization signal peptides, the cells were rinsed using PBS, placed in HBSS solution for fluorescence detection under the FITC channel using an inverted fluorescence microscope. The results were shown in Figure 5. phosphoenolpyruvate optical probes were able to localize to subcellular organelles including cytosol, extracellular membrane, nucleus, endoplasmic reticulum, mitochondria, nuclear exclusion, etc., by fusing with different specific localization signal peptides. Fluorescence was present in different subcellular structures, and the distribution and intensity of fluorescence varied.

Thirty-six hours after transfection of 293 cells with optical probe plasmids expressed in the cytosol, the cells were rinsed with PBS, placed in an HBSS solution, and the change in the ratio of the fluorescence intensity at an excitation of 420 nm and an emission of 528 nm to that at an excitation of 485 nm and an emission of 528 nm was detected in a 30-minute period. As shown in Figure 6, 5 mM Oxalate was added, and the detection was continued for 30 minutes. The 485/420 ratio of the sample with Oxalate gradually increased, reaching up to 3.5 times the initial value, while the 485/420 ratio of the control group without Oxalate remained at 0.437.

### Example 10: High-Throughput Compound Screening Based on the Optical Probe in Living Cells

In this example, HeLa cells expressing 254/255-TtPFK-I253K/Q254L/R255L/G256F-cpYFP-Y1S in the cytosol were used for high-throughput compound screening.

Transfected 293 cells were rinsed with PBS, placed in HBSS solution (without phosphoenolpyruvate) and treated for 1 hour, and then treated with a 10 µM compound for 1 hour. Phosphoenolpyruvate was added dropwise in each sample, respectively. Changes in the ratio of fluorescence intensity at 420 nm excitation and 528 nm emission to that of 485 nm excitation and 528 nm emission were recorded using a microplate reader. Samples that were not treated with any compounds were used as controls for normalization. The results were shown in Figure 7. Of the 2000 compounds used, the vast majority had minimal effects on phosphoenolpyruvate's entry into cells. Twenty-three compounds were able to increase the cellular uptake of phosphoenolpyruvate, and other seven compounds were able to decrease the cellular uptake of phosphoenolpyruvate significantly.

### Example 11: Quantitative Detection of phosphoenolpyruvate in Blood with Optical Probes

In this example, purified probe 254/255-TtPFK-S216I-cpYFP with a Kd of 0.24 µM was used to analyze phosphoenolpyruvate in the blood supernatants of mice and humans.

After 10 min of treatment by mixing 254/255-TtPFK-S216I-cpYFP with diluted blood supernatants, the ratio of fluorescence intensity at 420 nm excition and 528 nm emission to that of 485 nm excition and 528 nm emission was measured using a microplate reader. Results were shown in Figure 8, phosphoenolpyruvate levels were around 91 µM in mouse blood and 18 µM in human blood.

It is known from the above examples that the phosphoenolpyruvate optical probes provided in the invention had relatively small protein molecular weight and was prone to maturation, had large dynamic change in fluorescence, excellent specificity, and ability to be expressed in cells by gene manipulation methods, which could locate, quantitatively detect phosphoenolpyruvate in real time inside and outside cells; and enables high-throughput compound screening.

### Other Embodiments

The specification describes several embodiments. It is understood, however, that the various improvements learned by those skilled in the art not to deviate from the conception and scope of the invention by reading these instructions shall also be included within the scope of the attached claims.

### Sequences

1>TtPFK (1-322 full length) 
MKRIGVFTSGGDAPGMNAAIRAVVRQAHALGVEVIGIRRGYAGMIQGEMVP
LGVRDVANIIQRGGTILLTARSQEFLTEEGRAKAYAKLQAAGIEGLVAIGGDGTFR
GALFLVEEHGMPVVGVPGTIDNDLYGTDYTIGFDTAVNTALEAIDRIRDTAASHER
VFFIEVMGRHAGFIALDVGLAGGAEVIAVPEEPVDPKAVAEVLEASQRRGKKSSIV
VVAEGAYPGGAAGLLAAIREHLQVEARVTVLGHIQRGGSPTAKDRILASRLGAPA
VEALVGGASGVMVGEVEGEVDLTPLKEAVERRKDINRALLRLSQVLAL
2>cpYFP 
YNSDNVYIMADKQKNGIKANFKIRHNVEDGSVQLADHYQQNTPIGDGPVLLP
DNHYLSFQSVLSKDPNEKRDHMVLLEFVTAAGITLGMDELYNVDGGSGGTGSK
GEELFTGVVPILVELDGDVNGHKFSVSGEGEGDATYGKLTLKLICTTGKLPVPWP
TLVTTLGYGLKCFARYPDHMKQHDFFKSAMPEGYVQERTIFFKDDGNYKTRAEV
KFEGDTLVNRIELKGIDFKEDGNILGHKLEYN
3>cpmOrange 
VSERMYPEDGVLKSEIKKGLRLKDGGHYAAEVKTTYKAKKPVQLPGAYIVDI
KLDIVSHNEDYTIVEQCERAEGRHPTGGRDELYKGGTGGSLVSKGEEDNMAIIKE
FMRFKVHMEGSVNGHEFEIEGEGEGRPYEAFQTAKLKVTKGGPLPFAWDILSPQ
FTYGSKAYIKHPADIPDYFKLSFPEGFRWERVMNFEDGGIIHVNQDSSLQDGVFI
YKVKLRGTNFPPDGPVMQKKTMGWEA
4>cpm Kate 
MGGRSKKPAKNLKMPGVYYVDRRLERIKEADKETYVEQHEVAVARYCDLP
SKLGHKLNGGTGGSMVSKGEELIKENMHMKLYMEGTVNNHHFKCTSEGEGKPY
EGTQTMRIKVVEGGPLPFAFDILATSFMYGSKTFINHTQGIPDFFKQSFPEGFTW
ERVTTYEDGGVLTATQDTSLQDGCLIYNVKIRGVNFPSNGPVMQKKTLGWEAST
EMLYPADGGLEGRSDMALKLVGGGHLICNLKTTYRSKK
5>mCherry 
MVSKGEEDNMAIIKEFMRFKVHMEGSVNGHEFEIEGEGEGRPYEGTQTAKL
KVTKGGPLPFAWDILSPQFMYGSKAYVKHPADIPDYLKLSFPEGFKWERVMNFE
DGGVVTVTQDSSLQDGEFIYKVKLRGTNFPSDGPVMQKKTMGWEASSERMYPE
DGALKGEIKQRLKLKDGGHYDAEVKTTYKAKKPVQLPGAYNVNIKLDITSHNEDY
TIVEQYERAEGRHSTGGMDELYK
6>cpGFP 
NVYIKADKQKNGIKANFKIRHNIEDGGVQLAYHYQQNTPIGDGPVLLPDNHY
LSVQSILSKDPNEKRDHMVLLEFVTAAGITLGMDELYKGGTGGSMVSKGEELFT
GVVPIQVELDGDVNGHKFSVSGEGEGDATYGKLTLKFICTTGKLPVPWPTLVTTL
TYGVQCFSRYPDHMKQHDFFKSAMPEGYIQERTIFFKDDGNYKTRAEVKFEGDT
LVNRIELKGIDFKEDGNILGHKLEYN
7>cpBFP 
NVYIKADKQKNGIKANFKIRHNIEGGGVQLAYHYQQNTPIGDGPVLLPDNHY
LSVQSILSKDPNEKRDHMVLLEFVTAAGITLGMDELYKGGTGGSESMVSKGEELF
TGVVPIQVELDGDVNGHKFSVSGEGEGDATYGKLTLKFICTTGKLPVPWPTLVTT
LSHGVQCFSRYPDHMKQHDFFKSAMPGGYIQERTIFFKDDGNYKTRAEVKFEGD
TLVNRIELKGIDFKEDGNILGHKLEYN
8>mKate 
MSELITENMHMKLYMEGTVNNHHFKCTSEGEGKPYEGTQTMRIKVVEGGPL
PFAFDILATSFMYGSKTFINHTQGIPDFFKQSFPEGFTWERVTTYEDGGVLTATQ
DTSLQDGCLIYNVKIRGVNFPSNGPVMQKKTLGWEASTEMLYPADGGLEGRAD
MALKLVGGGHLICNLKTTYRSKKPAKNLKMPGVYYVDRRLERIKEADKETYVEQH
EVAVARYCDLPSKLGHKLN
9>cpmApple 
VSERMYPEDGALKSEIKKGLRLKDGGHYAAEVKTTYKAKKPVQLPGAYIVDI
KLDIVSHNEDYTIVEQCERAEGRHSTGGMDELYKGGTGGSLVSKGEEDNMAIIKE
FMRFKVHMEGSVNGHEFEIEGEGEGRPYEAFQTAKLKVTKGGPLPFAWDILSPQ
FMYGSKAYIKHPADIPDYFKLSFPEGFRWERVMNFEDGGIIHVNQDSSLQDGVFI
YKVKLRGTNFPPDGPVMQKKTMGWEA
10>254/255-TtPFK-cpYFP 
MKRIGVFTSGGDAPGMNAAIRAVVRQAHALGVEVIGIRRGYAGMIQGEMVP
LGVRDVANIIQRGGTILLTARSQEFLTEEGRAKAYAKLQAAGIEGLVAIGGDGTFR
GALFLVEEHGMPVVGVPGTIDNDLYGTDYTIGFDTAVNTALEAIDRIRDTAASHER
VFFIEVMGRHAGFIALDVGLAGGAEVIAVPEEPVDPKAVAEVLEASQRRGKKSSIV
VVAEGAYPGGAAGLLAAIREHLQVEARVTVLGHIQYNSDNVYIMADKQKNGIKAN
FKIRHNVEDGSVQLADHYQQNTPIGDGPVLLPDNHYLSFQSVLSKDPNEKRDHM
VLLEFVTAAGITLGMDELYNVDGGSGGTGSKGEELFTGVVPILVELDGDVNGHKF
SVSGEGEGDATYGKLTLKLICTTGKLPVPWPTLVTTLGYGLKCFARYPDHMKQH
DFFKSAMPEGYVQERTIFFKDDGNYKTRAEVKFEGDTLVNRIELKGIGFKEDGNIL
GHKLEYNRGGSPTAKDRILASRLGAPAVEALVGGASGVMVGEVEGEVDLTPLKE
AVERRKDINRALLRLSQVLAL
11>Nucleic acid sequence of 254/255-TtPFK-cpYFP 
atgaaacgcattggcgtgtttaccagcggtggtgatgcgcctggtatgaatgcggcgattcgtgcggttgttcgtcag
gcgcatgcgttaggcgttgaagtgattggcattcgccgtggctatgcgggtatgattcagggtgaaatggtgccttta
ggcgtgcgtgatgtggcgaacattattcaacgcggcggcaccattttattaaccgcgcgcagccaagaatttctga
ccgaagaaggccgcgcaaaagcgtatgcgaaactgcaggcggcgggtattgaaggtctggtggcgattggtgg
tgatggaacctttcgtggcgcgctgtttctggtggaagaacatggcatgccggttgtgggtgtgcctggaaccattga
taacgatctgtacggcaccgattataccattggctttgataccgcggttaacacagccctggaagcgattgatcgca
ttcgtgataccgcggcgtcacatgaacgcgtgtttttcattgaagtgatgggccgccatgcgggttttattgcgctggat
gttggcttagcaggcggtgcggaagttattgcggtgccggaagaacctgtggacccgaaagcggtggcggaagt
tttagaagcgagccaacgccgtggcaaaaaaagcagcattgtggtggttgcggaaggtgcatatcctggtggcg
cggcgggtttattagcggcgattcgcgaacatctgcaggttgaagcgcgcgttaccgtgttaggccatattcagtac
aacagcgacaacgtctatatcatggccgacaagcagaagaacggcatcaaggccaacttcaagatccgccac
aacgtcgaggacggcagcgtgcagctcgccgaccactaccagcagaacacccccatcggcgacggccccgt
gctgctgcccgacaaccactacctgagcttccagtccgtcctgagcaaagaccccaacgagaagcgcgatcac
atggtcctgctggagttcgtgaccgccgccgggatcactctcggcatggacgagctgtacaacgtggatggcggt
agcggtggcaccggcagcaagggcgaggagctgttcaccggggtggtgcccatcctggtcgagctggacggc
gacgtaaacggccacaagttcagcgtgtccggcgagggcgagggcgatgccacctacggcaagctgaccctg
aagctgatctgcaccaccggcaagctgcccgtgccctggcccaccctcgtgaccaccctcggctacggcctgaa
gtgcttcgcccgctaccccgaccacatgaagcagcacgacttcttcaagtccgccatgcccgaaggctacgtcca
ggagcgcaccatcttcttcaaggacgacggcaactacaagacccgcgccgaggtgaagttcgagggcgacac
 
cctggtgaaccgcatcgagctgaagggcatcggcttcaaggaggacggcaacatcctggggcacaagctgga
gtacaaccgtggtggtagccctaccgcgaaagatcgtattctggcgagccgtttaggtgcacctgcggttgaagcg
ttagttggtggtgcgagcggcgttatggttggtgaagtggaaggcgaagtggatctgaccccgctgaaagaagca
gttgaacgccgcaaagatattaaccgcgcgttactgcgcttatcacaggtgctggcgtta
12>254/255-TIPFK-1253K/Q254L/R255L/G256F-cpYFP-Y1S 
MKRIGVFTSGGDAPGMNAAIRAVVRQAHALGVEVIGIRRGYAGMIQGEMVP
LGVRDVANIIQRGGTILLTARSQEFLTEEGRAKAYAKLQAAGIEGLVAIGGDGTFR
GALFLVEEHGMPVVGVPGTIDNDLYGTDYTIGFDTAVNTALEAIDRIRDTAASHER
VFFIEVMGRHAGFIALDVGLAGGAEVIAVPEEPVDPKAVAEVLEASQRRGKKSSIV
VVAEGAYPGGAAGLLAAIREHLQVEARVTVLGHKLSNSDNVYIMADKQKNGIKAN
FKIRHNVEDGSVQLADHYQQNTPIGDGPVLLPDNHYLSFQSVLSKDPNEKRDHM
VLLEFVTAAGITLGMDELYNVDGGSGGTGSKGEELFTGVVPILVELDGDVNGHKF
SVSGEGEGDATYGKLTLKLICTTGKLPVPWPTLVTTLGYGLKCFARYPDHMKQH
DFFKSAMPEGYVQERTIFFKDDGNYKTRAEVKFEGDTLVNRIELKGIGFKEDGNIL
GHKLEYNLFGSPTAKDRILASRLGAPAVEALVGGASGVMVGEVEGEVDLTPLKE
AVERRKDINRALLRLSQVLAL
13> Nucleic acid sequence of 254/255-TtPFK-I253K/Q254L/R255L/G256F-cpYFP-Y1S

atgaaacgcattggcgtgtttaccagcggtggtgatgcgcctggtatgaatgcggcgattcgtgcggttgttcgtcag
gcgcatgcgttaggcgttgaagtgattggcattcgccgtggctatgcgggtatgattcagggtgaaatggtgccttta
ggcgtgcgtgatgtggcgaacattattcaacgcggcggcaccattttattaaccgcgcgcagccaagaatttctga
ccgaagaaggccgcgcaaaagcgtatgcgaaactgcaggcggcgggtattgaaggtctggtggcgattggtgg
tgatggaacctttcgtggcgcgctgtttctggtggaagaacatggcatgccggttgtgggtgtgcctggaaccattga
taacgatctgtacggcaccgattataccattggctttgataccgcggttaacacagccctggaagcgattgatcgca
ttcgtgataccgcggcgtcacatgaacgcgtgtttttcattgaagtgatgggccgccatgcgggttttattgcgctggat
gttggcttagcaggcggtgcggaagttattgcggtgccggaagaacctgtggacccgaaagcggtggcggaagt
tttagaagcgagccaacgccgtggcaaaaaaagcagcattgtggtggttgcggaaggtgcatatcctggtggcg
cggcgggtttattagcggcgattcgcgaacatctgcaggttgaagcgcgcgttaccgtgttaggccataagttgtcg
aacagcgacaacgtctatatcatggccgacaagcagaagaacggcatcaaggccaacttcaagatccgccac
aacgtcgaggacggcagcgtgcagctcgccgaccactaccagcagaacacccccatcggcgacggccccgt
gctgctgcccgacaaccactacctgagcttccagtccgtcctgagcaaagaccccaacgagaagcgcgatcac
atggtcctgctggagttcgtgaccgccgccgggatcactctcggcatggacgagctgtacaacgtggatggcggt
agcggtggcaccggcagcaagggcgaggagctgttcaccggggtggtgcccatcctggtcgagctggacggc
gacgtaaacggccacaagttcagcgtgtccggcgagggcgagggcgatgccacctacggcaagctgaccctg
aagctgatctgcaccaccggcaagctgcccgtgccctggcccaccctcgtgaccaccctcggctacggcctgaa
gtgcttcgcccgctaccccgaccacatgaagcagcacgacttcttcaagtccgccatgcccgaaggctacgtcca
ggagcgcaccatcttcttcaaggacgacggcaactacaagacccgcgccgaggtgaagttcgagggcgacac
cctggtgaaccgcatcgagctgaagggcatcggcttcaaggaggacggcaacatcctggggcacaagctgga
gtacaatttgtttggtagccctaccgcgaaagatcgtattctggcgagccgtttaggtgcacctgcggttgaagcgtta
gttggtggtgcgagcggcgttatggttggtgaagtggaaggcgaagtggatctgaccccgctgaaagaagcagtt
gaacgccgcaaagatattaaccgcgcgttactgcgcttatcacaggtgctggcgtta

## Claims

1. A phosphoenolpyruvate binding protein variant, wherein, the variant:
(a) has the sequence shown in SEQ ID NO:1 and having mutations at 1, 2, 3, 4, 5 or more sites selected from the group consisting of: I253, Q254, R255, G256, G257, R21, V54, R55, V57, A58, N59, I60, R72, R212, G213, K215, S216, L320, wherein the mutations comprise modifications, substitutions or deletions of amino acids, or
(b) is a sequence having at least 70% sequence identity with (a) and having the mutation described in (a) and retaining the ability to bind to phosphoenolpyruvate,
preferably, the mutations described in (a) include mutations at sites selected from any one or more of the groups consisting of:

2. A phosphoenolpyruvate optical probe, comprising a phosphoenolpyruvate-sensitive polypeptide and an optically active polypeptide, wherein the optically active polypeptide is located in the sequence of the phosphoenolpyruvate-sensitive polypeptide, and
the phosphoenolpyruvate-sensitive polypeptide has:
(1) the sequence of SEQ ID NO: 1, or a sequence having at least 70% sequence identity with it and retaining the binding activity to phosphoenolpyruvate,
(2) a functional variant of the sequence of SEQ ID NO: 1, which has a mutation within 5 amino acids from the junction with the optically active polypeptide, and/or has mutations at 1, 2, 3, 4, 5 or more sites selected from the group consisting of: R21, V54, R55, V57, A58, N59, I60, R72, R212, G213, K215, S216, L320, or
(3) a sequence having at least 70% sequence identity to the sequence described in (2), having the mutations described in (2), and retaining the sensitivity to phosphoenolpyruvate,
preferably, (2) is the sequence of the phosphoenolpyruvate-binding protein variant according to claim 1.

3. The optical probe according to claim 2, wherein the optically active polypeptide is located in residues 68-71, 168-173 and/or 253-257 of the phosphoenolpyruvate-sensitive polypeptide or substitutes the residues therein.

4. The optical probe according to claim 2, wherein,
the optically active polypeptide is located at any one or more of the following sites of the phosphoenolpyruvate-sensitive polypeptide: 68/69, 68/70, 69/70, 68/71, 69/71, 70/71, 168/169, 168/170, 169/170, 168/171, 169/171, 170/171, 168/172, 169 /172, 170/172, 171/172, 168/173, 169/173, 170/173, 171/173, 172/173, 253/254, 253/255, 254/255, 253/256, 254/256, 255/256, 253/257, 254/257, 255/257 and/or 256/257,
further preferably,
the optically active polypeptide is located at any one or more of the following sites of the phosphoenolpyruvate-sensitive polypeptide: 69/71, 170/171, 171/172, 254/255, 254/256 and 255/257,
further preferably, wherein, the optically active polypeptide is a fluorescent protein or a functional variant thereof,
preferably,
the fluorescent protein has the sequence shown in any one of SEQ ID NO: 2-9,
the functional variant of the fluorescent protein has a mutation within 3 amino acids from the junction with the optically active polypeptide; preferably, the functional variant of the fluorescent protein has a mutation at the first amino acid corresponding to SEQ ID NO: 2, and the mutation is preferably selected from Y1S, Y1G, Y1N, Y1T, Y1P, Y1A, Y1E, Y1H,
the functional variant of the fluorescent protein has a mutation at amino acid 246 corresponding to SEQ ID NO: 2, and the mutation is preferably selected from N246F, N246C, N246W or N246H,
further preferably, in the optical probe, the phosphoenolpyruvate-sensitive polypeptide is as shown in SEQ ID NO: 1, and has one or more of the following mutations: I253V, I253N, I253F, I253C, I253E, I253G, I253S, I253K, I253H, I253D, I253P, I253L, I253R, I253T, I253Q, Q254L, Q254V, Q254A, Q254R, Q254P, Q254M, Q254K, Q2541, R255L, R2551, R255F, G256H, G256F, G256Y, G256V, G257L, R21K, V54G, V54K, V54Q, R55S, V57M, A58G, N59D, N59H, N59E, I60W, R72A, R2121, R212E, R212M, G213T, K215H, K215D, K215E, S216D, S216L, S216Y, S2161, S216M, S216F, S216Q, L320Y, L320N and/or L320F, the optically active polypeptide is as shown in SEQ ID NO: 2-9 or is a variant having one or more of the following mutations: Y1S, Y1G, Y1N, Y1T, Y1P, Y1A, Y1E, Y1H, N246F, N246C, N246W, and the optically active polypeptide is located at the 254/255 or 254/256 of the phosphoenolpyruvate-sensitive polypeptide.

5. A nucleic acid molecule, comprising:
(a) the coding sequence of the optical probe as described in any one of claims 2-4,
(b) the complementary sequence of (a).

6. A nucleic acid construct comprising the nucleic acid molecule according to claim 5,
preferably, the nucleic acid construct is a cloning vector, an expression vector, or a recombinant vector.

7. A host cell, wherein the host cell:
(1) expresses the optical probe according to any one of claims 2-4;
(2) comprises the nucleic acid molecule according to claim 5; or
(3) comprises the nucleic acid construct according to claim 6.

8. A detection kit, comprising:
(1) the optical probe according to any one of claims 2-4,
(2) the nucleic acid sequence according to claim 5,
(3) the nucleic acid construct according to claim 6, or
(4) the host cell according to claim 7,
the detection kit optionally further comprises additional reagents required for detection of phosphoenolpyruvate by the optical probe,
preferably, the detection kit further comprises one or more reagents selected from the group consisting of: buffer, culture medium, and phosphoenolpyruvate standard.

9. A method for preparing the optical probe according to any one of claims 2-4, comprising: providing the host cell according to claim 7, culturing the host cell under conditions for the expression of the optical probe, and isolating the optical probe.

10. Use of the optical probe according to any one of claims 2-4, the nucleic acid sequence according to claim 5, the nucleic acid construct according to claim 6, or the host cell according to claim 7 for detecting phosphoenolpyruvate in a sample, screening compounds, and localizing phosphoenolpyruvate intracellularly/extracellularly,
preferably,
detecting phosphoenolpyruvate in a sample comprises: contacting the optical probe or the host cell with the sample, detecting the optical change of the optically active polypeptide, and detecting phosphoenolpyruvate in the sample according to the optical change of the optically active polypeptide,
screening compounds comprises: contacting the optical probe or the host cell with a candidate compound in a system comprising phosphoenolpyruvate, detecting the optical change of the optically active polypeptide, and screening the compound according to the optical change of the optically active polypeptide; preferably, screening the compound comprises: contacting the host cell with the candidate compound in a system comprising phosphoenolpyruvate, and the optical change of the optically active polypeptide indicates whether the candidate compound regulates the uptake of phosphoenolpyruvate by cells,
localizing phosphoenolpyruvate intracellularly/extracellularly comprises: contacting a system comprising phosphoenolpyruvate with the optical probe or the host cell, and detecting the optical change of the optically active polypeptide,
further preferably, the system is a solution, a cell or a subcellular system.
